(19) Europäisches Patentamt
European Patent Office
Office européen des brevets

(11) Publication number: **0 032 432**
**B1**

(12) # EUROPEAN PATENT SPECIFICATION

(45) Date of publication of patent specification: **24.07.85**

(21) Application number: **81300078.3**

(22) Date of filing: **08.01.81**

(51) Int. Cl.4: **C 07 D 295/14,**
C 07 D 307/42,
C 07 D 333/16,
C 07 D 333/12, A 61 K 31/557
// C07C177/00

(54) **Prostanoid compounds and their preparation and pharmaceutical formulations.**

(30) Priority: **09.01.80 GB 8000697**
**09.01.80 GB 8000698**

(43) Date of publication of application:
**22.07.81 Bulletin 81/29**

(45) Publication of the grant of the patent:
**24.07.85 Bulletin 85/30**

(84) Designated Contracting States:
**AT BE CH DE FR IT LI NL SE**

(56) References cited:
**FR-A-2 430 934**

(73) Proprietor: **GLAXO GROUP LIMITED**
**Clarges House 6-12 Clarges Street**
**London W1Y 8DH (GB)**

(72) Inventor: **Collington, Eric William**
**103 Warren Way**
**Digswell, Welwyn Hertfordshire (GB)**
Inventor: **Hallett, Peter**
**117 Snellsmead**
**Buntingford Hertfordshire (GB)**
Inventor: **Wallis, Christopher John**
**40 Cherry Drive**
**Royston Hertfordshire (GB)**
Inventor: **Bradshaw, John**
**22 Whitely Close**
**Dane End Ware, Hertfordshire (GB)**

(74) Representative: **Skailes, Humphrey John et al**
**Frank B. Dehn & Co. Imperial House 15-19**
**Kingsway**
**London WC2B 6UZ (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 032 432 B1

**Description**

Prostaglandins are a class of naturally occurring cyclopentane derivatives which are biologically active in many physiological systems and they and substances which antagonise their effects are therefore of considerable interest in both human and veterinary medicine.

In view of the activity found in the natural prostaglandins, considerable effort has been directed towards the preparation of synthetic analogues. Many such compounds have been described, and in general it has been reported that these compounds possess activity within the same spectrum as the natural compounds. The synthetic compounds can however have increased selectivity of action, longer duration of activity or different potency, and in some cases they can antagonise the activity of natural prostaglandins.

In most of the synthetic prostanoids previously reported, the side chains have been attached to the cyclopentane ring via carbon atoms, as in the natural prostaglandin structure. We have now found a new class of prostanoid compounds in which the α-side chain has the same or similar structure to that of the natural compounds, while the β-side chain is attached to the ring via a nitrogen atom and the ring is also substituted by certain aralkoxy or heteroaralkoxy groups. Compounds in this class have shown prostanoid activity in our tests and in particular they inhibit blood platelet aggregation and have bronchodilatatory action.

The invention provides prostanoids of the general formula (1)

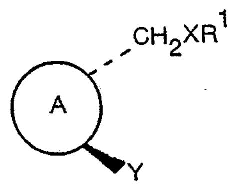

(1)

in which

A represents

(a) 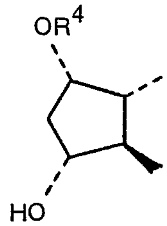 or (b) 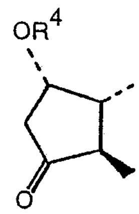

X is cis or trans —CH=CH— or —$(CH_2)_2$—;

$R^1$ is straight or branched $C_{1-7}$ alkyl bearing as a terminal substituent —$COOR^{10}$ where $R^{10}$ is a hydrogen atom, $C_{1-6}$ alkyl or $C_{7-10}$ phenalkyl (e.g. benzyl);

Y represents a saturated heterocyclic amino group which has 5—8 ring members and (a) optionally contains in the ring —O—, —S—, —$SO_2$, —$NR^{14}$— (where $R^{14}$ is a hydrogen atom, $C_{1-7}$ alkyl or phenalkyl having a $C_{1-4}$ alkyl portion), >C(OH)$R^6$ (where $R^6$ is a hydrogen atom, $C_{1-7}$ alkyl, phenyl, or phenalkyl having a $C_{1-4}$ alkyl portion); and/or (b) is optionally substituted by one or more $C_{1-4}$ alkyl groups;

$R^4$ is (i) phenalkyl (having a $C_{1-3}$ alkyl portion and the phenyl portion being substituted by $C_{1-3}$ alkylthio, $C_{1-3}$ alkylsulphinyl, $C_{1-3}$ alkylsulphonyl, $C_{1-3}$ alkanoylamino, benzoylamino, phenylalkyl having a $C_{1-3}$ alkyl portion, aminosulphonyl, (the amino group being optionally substituted by one or two $C_{1-3}$ alkyl groups), $C_{1-3}$ alkanoylaminosulphonyl (the amino group being optionally substituted by $C_{1-3}$ alkyl), phenylsulphonyl (the phenyl portion being optionally substituted by $C_{1-3}$ alkyl), nitro, tetrazol-5-yl, phenyl substituted by $R^5$ (where $R^5$ is $C_{1-4}$ alkyl, $C_{1-4}$ alkoxy, halogen or phenyl), or thienyl);

or (ii) the group:

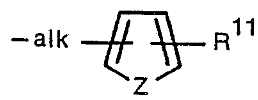

where

alk is $C_{1-3}$ alkylene;

Z is O or S;

$R^{11}$ is a hydrogen atom; $C_{1-6}$ alkyl; $C_{1-6}$ alkoxy; phenyl or phenylalkoxy or phenylalkyl having a $C_{1-3}$ alkyl portion (the phenyl portion in each case being optionally substituted by $C_{1-3}$ alkyl, $C_{1-3}$ alkoxy or halogen); phenoxy; $C_{5-7}$ cycloalkyl; halogen or nitro;

and the physiologically acceptable salts thereof.

The formulae used herein are to be understood to depict either or both optical isomers of each of the compounds concerned as well as mixtures of the isomers, including racemates, even though the precise structure as set out only relates to one optical isomer.

Compounds having the ring type (b) are particularly important.

In the group $—CH_2XR^1$, the alkyl portion of the group $R^1$ may for example contain 2—5 carbon atoms (straight or branched) and is preferably $—(CH_2)_3COOR^{10}$. $R^{10}$ is preferably a hydrogen atom or methyl, particularly hydrogen.

When $R^1$ is terminally substituted by —COOH, the compounds are capable of salt formation with bases, examples of suitable salts being alkali metal (e.g. sodium and potassium), alkaline earth metal (e.g. calcium), ammonium, substituted ammonium (e.g. tromethamine or dimethylaminoethanol), piperazine, morpholine, piperidine and tertiary amine (e.g. triethylamine) salts.

X is preferably $—CH_2CH_2—$ or cis —CH=CH—, particularly the latter.

The types of heterocyclic Y group which are generally preferred are those in which the ring has 5—8 members and (a) optionally contains —O—, —S—, $—SO_2—$ or $—NR^{14}—$ and/or (b) is optionally substituted by one or more $C_{1-4}$ alkyl (e.g. methyl) groups. The group may for example have a 5, 6 or 7-membered ring, e.g. pyrrolidino, piperidino, morpholino, piperazino, thiamorpholino, 1-dioxothiamorpholino, homomorpholino and hexamethyleneimino.

Examples of the optional substituents which may be present on a second nitrogen atom in the ring are methyl, ethyl and benzyl. The carbon atoms of the heterocyclic rings may for example be substituted by methyl or ethyl. The group $>C(OH)R^6$ may for example be present in a piperidino ring and when $R^6$ is other than hydrogen it may for example be methyl, ethyl or butyl.

Compounds in which Y is a morpholino, dioxothiamorpholino or piperidino group are particularly preferred.

The amino group in the group Y enables the compounds to form salts with inorganic or organic acids, e.g. hydrochlorides, sulphates, acetates, maleates and succinates.

When $R^4$ is phenalkyl, it is preferably a benzyl group substituted by $C_{1-3}$ alkylthio (e.g. methylthio, $C_{1-3}$ alkylsulphinyl (e.g. methylsulphinyl), $C_{1-3}$ alkanoylamino (e.g. acetylamino), $C_{1-3}$ alkylsulphonyl (e.g. methylsulphonyl), phenylsulphonyl, aminosulphonyl (e.g. dimethylaminosulphonyl), phenylalkyl (e.g. phenethyl or benzyl), phenyl substituted by the group $R^5$ (where $R^5$ represents $C_{1-4}$ alkyl (e.g. methyl), $C_{1-4}$ alkoxy (e.g. methoxy), halogen (e.g. chlorine), or phenyl) or thienyl.

Particularly preferred substituents of this type are phenylalkyl (e.g. phenethyl or benzyl), or, more particularly, phenyl substituted by $C_{1-3}$ alkoxy (e.g. methoxy) or $C_{1-3}$ alkyl (e.g. methyl).

When $R^4$ is a group of the type (ii) the alkylene group is preferably methylene, and Z is preferably S. Where Z is S, the thienyl group is preferably substituted by $C_{1-3}$ alkyl (e.g. methyl), phenyl, optionally substituted by $C_{1-3}$ alkoxy, (e.g. methoxy), $C_{5-7}$ cycloalkyl (e.g. cyclohexyl), halogen (e.g. bromine) or phenalkyl (e.g. phenethyl), and where Z is 0 the furanyl group is preferably substituted by an aryl, e.g. phenyl group. Particularly preferred groups of this type are phenylthienylalkyl and alkoxyphenylthienylalkyl.

A particularly preferred group of compounds has the formula 1(b) where:

X is cis —CH=CH—,

$R^1$ is $—(CH_2)_3COOH$,

Y is morpholino, piperidino, or 1-dioxothiamorpholino, and

$R^4$ is benzyl substituted by phenethyl, benzyl, methoxyphenyl or methylphenyl, or is methoxyphenylthienylmethyl.

As indicated above, our tests have shown that compounds of formula (1) inhibit blood platelet aggregation and/or have bronchodilatatory activity. The test we have used for bronchodilatation is as described by K. M. Lulich, *et al* in British Journal of Pharmacology *58*, 71—79, (1976) except guinea-pig lung is used instead of cat lung. The test for inhibition of platelet aggregation is as described by G. V. Born in Nature *194*, 927—929 (1962) except collagen is used instead of ADP as the pro-aggregatory agent.

The compounds are thus of interest in the treatment of asthma and as antithrombotic agents for use in the treatment and prevention of cardiovascular diseases or conditions such as arteriosclerosis, atherosclerosis and myocardial infarcts. They may be formulated for use in conventional manner, with one or more pharmaceutical carriers.

For oral administration, the pharmaceutical composition may take the form of, for example, tablets, capsules, powders, solutions, syrups, or suspensions prepared by conventional means with acceptable excipients.

The compounds may be formulated for parenteral administration by bolus injections or continuous infusion. Formulations for injections may be presented in unit dosage form in ampoules, or in multi-dose containers, with an added preservative. The compositions may take such forms as suspensions, solutions, or emulsions in oily or aqueous vehicles, and may contain formulatory agents such as suspending, stabilising and/or dispersing agents. Alternatively, the active ingredient may be in powder form for reconstitution before use with a suitable vehicle, e.g. sterile pyrogen-free water.

For administration by inhalation the compounds are conveniently delivered in the form of an aerosol spray presentation from pressurised packs or a nebuliser, or as a cartridge from which the powdered

composition may be inhaled with the aid of a suitable device. In the case of a pressurised aerosol the dosage unit may be determined by providing a valve to deliver a metered amount.

For use as antithrombotic agents, the compounds are preferably administered orally, for example in amounts of 0.1 to 10 mg/kg body weight, 1 to 4 times daily. For use in the treatment of asthma, the compounds may also be administered orally in amounts of 0.1 to 10 mg/kg body weight, 1 to 4 times daily; preferably however they are administered by inhalation in the form of aerosols or solutions for nebulisers, at doses varying from 0.3 to 30 mg, 1 to 4 times daily. The compounds may be used in combination with other anti-asthmatic agents. It will be appreciated that the precise dose administered will always depend on the age and condition of the patient.

The compounds of formula (1) may be prepared by selection and adaptation of methods known in prostanoid chemistry (see for example British Patent Specification 2028805A). Method (a) below is particularly important in forming certain prostanoids of the desired class, and other compounds in the class can be prepared from them by known techniques for example using one or more of methods (b) to (m) below.

The following reactions will frequently require the use of (or will conveniently be applied to) starting materials having protected functional groups (e.g. hydroxy). It is to be understood that references to the use of starting materials of a particular structure are intended to include starting materials having protected functional groups. Certain of the reactions described below are capable of affecting other groups in the starting material which are desired in the end product, and this must be taken into account when performing multi-stage reactions.

In the discussion below, the groups X and Y and the various R groups are as defined above except where otherwise indicated.

(a) Compounds of formula (2)

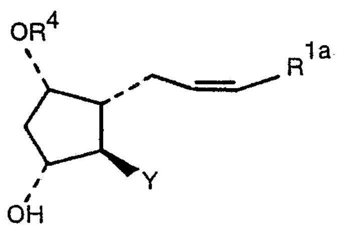

(2)

(where $R^{1a}$ is as defined above for $R^1$ where $R^{10}$ is a hydrogen atom) may be prepared by reacting lactols of formula (3) or their aldehyde isomers of formula (3a)

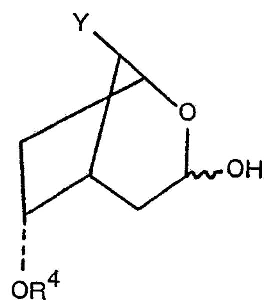

(3)

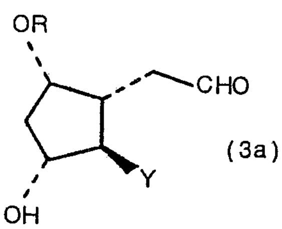

(3a)

with appropriate Wittig reagents, e.g. a phosphorane of formula $R^{12}_3P{=}CHR^{1a}$ (where $R^{12}$ is $C_{1-6}$ alkyl or aryl, e.g. monocyclic aryl such as phenyl), or a salt thereof, e.g. the potassium salt. Suitable reaction solvents include hydrocarbons (e.g. benzene and toluene), ethers (e.g. tetrahydrofuran), dialkylsulphoxides (e.g. dimethylsulphoxide), alcohols and halogenated hydrocarbons. The reaction may be carried out at any suitable temperature up to 50°C, preferably at room temperature.

The reaction is particularly suitable for the preparation of compounds in which $R^1$ is terminally substituted by —COOH (in salt form). Any hydroxy group in Y should preferably be in a protected state prior to this reaction. Suitable hydroxy protecting groups are described below. Any —$NH_2$ group present should also be protected, e.g. by t-butoxycarbonyl.

Except as regards the nature of $R^4$, this reaction is the same as process (a) of British Patent Specification 2028805A. The intermediates of formulae (3) and (3a) may thus be prepared by the methods described in that specification, using starting materials containing the desired $R^4$ group.

These starting materials may themselves be prepared by the same general methods as described in Specification 2028805A. However, when $R^4$ is an aralkyl group substituted by phenylmethyl, substituted phenyl or thienyl, it can be more convenient to introduce first an aralkyl $R^4$ group substituted by halo; after the formation of the intermediate lactone, this group can then be modified to form the desired $R^4$ group, for example by treatment with the appropriate aryl or benzyl zinc halide in the presence of a catalyst such as $Ni(PPH_3)_4$ or $Cl_2Pd$ $(PPh_3)_2$/diisobutylaluminium hydride in a solvent such as tetrahydrofuran at e.g. 10°-ambient temperature.

(b) Compounds of ring type (b) may be prepared by oxidising the corresponding hydroxy compound of ring type (a), for example with a $Cr^{VI}$ oxidising reagent, e.g. Jones reagent, at −10° to room temperature, preferably −10 —0°, in a solvent such as acetone. Other conventional methods can also be used, for example using dimethylsulphoxide and a suitable electrophilic reagent, such as acetyl bromide, oxalyl chloride, thionyl chloride, or dicyclohexylcarbodiimide in a hydrocarbon solvent such as toluene at low temperature e.g. −70°. With the latter reagent, the reaction is preferably carried out in the presence of trifluoro acetic acid or its pyridinium salt.

Other suitable reagents are N-chlorosuccinimidedimethylsulphide complex (used for example in a hydrocarbon solvent, such as toluene, e.g. at 0—5°), and pyridine-sulphur trioxide complex in dimethylsulphoxide (e.g. at 0° to room temperature).

When the α-side chain has a terminal —COOH group (i.e. when $R^{10}$ is hydrogen), better yields are sometimes obtained by prior protecting the carboxyl group, for example in the form of a trialkyl (e.g. trimethyl or triethyl) silyl ester.

Any other hydroxy group present should be protected in this reaction.

(c) Compounds in which $R^{10}$ is alkyl or phenalkyl can be prepared by esterification of the corresponding carboxylic acid (in which $R^{10}$ is hydrogen). Conventional esterification techniques may be used, reaction with a diazoalkane being preferred. The alkyl esters may also be formed by reaction with an appropriate alcohol in the presence of a mineral acid, e.g. hydrochloric or sulphuric acid.

(d) Compounds in which $R^1$ is terminally substituted by a —COOH group can be prepared by saponifying a corresponding ester, e.g. using KOH or NaOH in methanol.

(e) Compounds in which X is trans —CH=CH— may be prepared by isomerising the corresponding cis compound. The isomerisation may be effected by treatment with, for example, p-toluene sulphinic acid in dioxan (e.g. at reflux) or azobisisobutyronitrile and thiophenol, using for example a hydrocarbon solvent and any suitable temperature up to reflux. Where an oxo group is desired in the end product, it should be introduced after this reaction.

(f) Compounds in which X is $—(CH_2)_2—$ may be prepared by catalytic hydrogenation of a corresponding compound in which X is —CH=CH—. Conventional catalysts may be used, preferably palladium or platinum on carbon, in a suitable solvent (e.g. an alcohol such as methanol) e.g. at room temperature.

(g) Compounds of formula (1a) may be prepared by etherification of the corresponding hydroxy compound (in which $R^4$ represents hydrogen), for example by reaction with an appropriate halide ($R^4$ Hal), for example by reaction at room temperature in the presence of a suitable base (e.g. sodium hydride) in a suitable solvent (e.g. dimethylformamide).

Any other hydroxy group present in the starting material (e.g. the ring hydroxy group) should be protected in this reaction.

Starting materials for this reaction may be prepared by the same general technique as described above for process (a), using intermediates in which the group $—OR^4$ is a protected hydroxy group and removing the protecting group prior to etherification.

Starting materials of the formula (4)

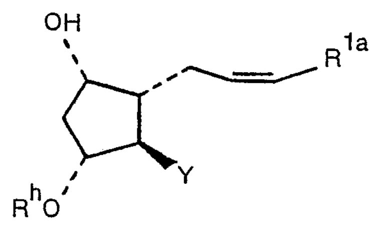

(4)

(where $R^{1a}$ is as defined above and $—OR^h$ represents a protected hydroxy group) may also be prepared by method (b) of British Patent Specification 2028805A.

(h) Compounds having ring type (a) can be prepared by removing the protecting group from the corresponding compound in which the ring hydroxy group is protected, for example by reduction or acid or alkaline hydrolysis. This is discussed below in connection with hydroxy group protection.

(i) Compounds in which $R^4$ is phenalkyl substituted by alkanoylamino may be prepared by acylation of the corresponding amine, e.g. with the appropriate acid anhydride in an organic base such as pyridine at 0°C.

(j) Compounds of formula (1a) in which $R^4$ is phenalkyl substituted by alkylsulphinyl or alkylsulphonyl may be prepared by oxidation of the corresponding alkylthio compound with a peracid for example peracetic acid at room temperature.

(k) Compounds of formula (1a) in which Y is a substituted amino group may be prepared by substitution of the corresponding compound in which Y is $—NH_2$.

This reaction may be performed by treating the starting material with a compound of the formula $JR^{13}J$, where J is a readily displaceable group (such as halo, e.g. iodo, or hydrocarbylsulphonyloxy, e.g. p-toluenesulphonyloxy) and $R^{13}$ is the appropriate divalent group (e.g. $—(CH_2)_2O(CH_2)_2—$). The reaction may be carried out in a solvent such as acetonitrile or methanol, in the presence of a suitable base, e.g. potassium carbonate or sodium bicarbonate.

Alternatively, the starting material may be reacted with an appropriate dialdehyde or diketone in the presence of a reducing agent. For example, reaction with glutardialdehyde gives a compound in which Y is piperidino. The reducing agents which may be used are those generally known for the reduction of imines, e.g. formic acid, or an alkali metal borohydride or cyanoborohydride (e.g. sodium borohydride or potassium cyanoborohydride, using an alcohol such as ethanol as solvent, suitably at room temperature, preferably at pH 4—6), or hydrogen in the presence of a metal catalyst, e.g. palladium.

The amines required as starting materials may be prepared by reduction of the corresponding azide, for example as described for process (1).

(l) Compounds of ring type (a) in which Y is $—NH_2$ and $R^{10}$ is hydrogen may be prepared by reducing the corresponding compound in which Y represents an azido group.

Compounds in which X is $—(CH_2)_2—$ may thus be prepared by catalytic hydrogenation, using for example platinum or palladium on carbon as the catalyst. However, when compounds in which X is —CH=CH— are required, selective reduction methods specific for the azide function should be used. Examples of suitable reagents are zinc and sodium dihydrogen phosphate in a suitable solvent (e.g. tetrahydrofuran); zinc and methanol/sulphuric acid; or triphenyl phosphine followed by methanol/sulphuric acid.

The azido starting materials required for this reaction may be prepared by methods analogous to those for preparing the compounds of formula (2), using reagents in which Y is azido. These methods are analogous to those of process (c) of British Patent Specification No. 2028805A.

(m) Salts of the compounds of formula (1) may be prepared by conventional methods, e.g. by treatment with an acid or (where $R^{10}$ is hydrogen) a base in a suitable solvent e.g. water or an organic solvent such as ether.

In the preparation of compounds of formula (1) the ring hydroxy group (or any other hydroxy group present) will often be protected and its liberation will frequently be the last step in the preparation. Conventional methods of protection may be used, protection in the form of t-butyldimethylsilyloxy or tetrahydropyranyloxy groups being preferred. These groups may be removed by acid hydrolysis. The group may also be protected in the form of an alkanoyloxy group having up to 7 carbon atoms, e.g. acetoxy. Such groups may be removed by alkaline hydrolysis.

The examples below illustrate the invention. The preparation of the intermediates required is described first.

The preparation of the following intermediates is described in British Patent Specification 2028805 A:

Intermediate 4:
(±)-3-endo-Hydroxy-2-exo-(4-morpholinyl)bicyclo[3.2.0]-heptan-6-one

Intermediate 5:
(3aα,4α,5β,6aα)-(±)-Hexahydro-5-hydroxy-4-(4-morpholinyl)-2H-cyclopenta(b)furan-2-one

Intermediate 6:
(3aα,4α,5β,6aα)-(±)-Hexahydro-4-(4-morpholinyl)-5-(tetrahydro-2H-pyran-2-yl)oxy-2H-cyclopenta(b)furan-2-one

Intermediate 7:
(3aα,4α,5β,6aα)-(±)-Hexahydro-4-(4-morpholinyl)-5-(tetrahydro-2H-pyran-2-yl)oxy-2H-cyclopenta(b)furan-2-ol

Intermediate 8:
[1α(Z),2β,3α,5α]-(±)-Methyl 7-[5-Acetoxy-2-(4-morpholinyl)-3-[(tetrahydro-2H-pyran-2-yl)oxy]cyclopentyl]-5-heptenoate

Intermediate 9:
[1α(Z),2β,3α,5α]-Methyl 7-[5-Hydroxy-2-(4-morpholinyl)-3-[(tetrahydro-2H-pyran-2-yl)oxy]cyclopentyl]-5-heptenoate

Intermediate 10:
[1α(Z),2β,3α,5α]-(±)-Methyl 7-[5-[4-Amino(phenylmethoxy)]-3-hydroxy-2-(4-morpholinyl)cyclopentyl]-5-heptenoate

Intermediate 11:
(±)-6-endo-(Phenylmethoxy)-8-anti-(4-thiomorpholinyl)-2-oxabicyclo[3.2.1]octan-3-one

Intermediate 41 :
[1α(Z),2β,3α,5α]-(±)-Methyl 7-[5-Hydroxy-2-(1-piperidinyl)-3-[(tetrahydro-2H-pyran-2-yl)ocy]cyclopentyl]-5-heptenoate

Temperatures are in °C. The following abbreviations are used:

TLC — thin layer chromatography, PE — petroleum ether (boiling at 40—60° unless otherwise stated), THF — tetrahydrofuran, EA — ethyl acetate, PTSA — p-toluenesulphonic acid monohydrate, DMF — dimethylformamide, DMSO — dimethylsulphoxide, Dibal — diisobutyalaluminium hydride.

Chromatography was carried out using silica gel. TLC was carried out using $SiO_2$. The following abbreviations illustrate the eluent used for the chromatography and TLC:

(A) 9:1 PE (b.p. 60—80°) — EA; (B) 4:1 PE (b.p. 60—80°) — EA; (C) 3:1 PE-ether; (D) ether-EA; (E) 9:1 EA—PE; (F) ether-PE; (G) 4:1 ether-PE; (H) 7:3 ether-PE; (I) 9:1 ether-methanol; (J) ether; (K) EA; (L) 19:1 ether-methanol; (M) 1:1 ether-PE (b.p. 60—80°); (N) 9:1 EA—PE (b.p. 60—80°); (O) EA—PE (b.p. 60—80°); (P) 19:1 EA-methanol; (Q) 95:5 ether-methanol; (R) 85:15 EA-methanol; (S) 3:1 EA-methanol; (T) 98:2 chloroform-methanol; (U) 95:5 EA-methanol; (V) 4:1 ether-methanol; (W) PE; (X) 9:1 EA-methanol; (Y) 7:3 EA—PE; (Z) 3:2 ether-PE; (AB) 1:1 EA—PE; (AC) 4:1 ether-isopentane; (AD) 39:1 ether-methanol; (AE) 4:1 ether-PE (b.p. 60—80°); (AF) ether-isopentane; (AG) chloroform; (AH) 97:3 chloroform-methanol; (AI) 7:3 EA—PE (b.p. 60—80°); (AJ) 85:15 ether-methanol; (AK) 97:3 ether-methanol; (AL) 99:1 ether-methanol; (AM) ether-methanol.

Intermediate 1

*1-(Bromomethyl)-4-(2-phenylethyl)benzene*

A solution of methyl 4-(2-phenethenyl)benzoate (5.4 g) in EA (200 ml) was hydrogenated over pre-reduced 10% Pd on charcoal (1 g) at atmospheric pressure. When hydrogen uptake had ceased the mixture was filtered and evaporated to give a solid (5.4 g).

A solution of the solid (4.7 g) in dry THF (20 ml) was added dropwise to a stirred suspension of $LiAlH_4$ (0.59 g) in dry THF (20 ml) at 0°. After 1 h Rochelle salt solution (50 ml) was added and the mixture extracted with EA (3 × 50 ml), washed with brine and dried ($MgSO_4$). $PBr_3$ (0.81 ml) was added to a solution of the dried extracts (3.4 g) in dry ether (75 ml) at 0° and the mixture was stirred for 0.5 h. Ice-water (70 ml) was added and stirring continued for a further 0.5 h. The aqueous phase was separated and extracted with ether (2 × 70 ml), washed with $NaHCO_3$ solution, brine and then dried ($MgSO_4$). Evaporation *in vacuo* gave the *title compound* as a solid (3.8 g) which was purified from PE (bp 60—80°) to give material of m.p. 52—3°.

Intermediate 2

*[4'Methyl-(1,1-biphenyl)-4-yl]methanol*

4-Methyl-(1,1'-biphenyl)-4-carboxylic acid, methyl ester (1.43 g) in ether (25 ml) and THF (25 ml) was added over 5 min to $LiAlH_4$ (420 mg) in ether (25 ml). The mixture was stirred at room temperature for 1 h and then cooled in ice. Aqueous NaOH (1M, 2.1 ml) was added and after stirring (15 min) excess anhydrous $Na_2SO_4$ was added. The mixture was filtered and the filtrate evaporated to give a solid. Crystallisation from cyclohexane-methanol gave the *title compound* (1.04 g) m.p. 128—31°.

Intermediate 3

*4-Bromomethyl-4'-methyl (1,1'-biphenyl)*

To a cold (0°) solution of Intermediate 2 (0.917 g) in dry $CH_2Cl_2$ (14 ml) was added $PBr_3$ (0.29 ml). After stirring for 1 h at 0°, 8% $NaHCO_3$ solution (30 ml) was added and the layers separated. The aqueous layer was extracted with $CH_2Cl_2$ (2 × 30 ml), dried ($MgSO_4$) and evaporated to give a solid (0.99 g). Crystallisation from PE (b.p. 60—80°) afforded the *title compound* (0.91 g) m.p. 100—102°.

Intermediate 12

*(±)-7-anti-(4-Morpholinyl)-5-endo-[tetrahydro-2H-pyran-2-yl)oxy]bicyclo[2.2.1]heptan-2-one*

Morpholine (76 ml) was added dropwise over 15 mins to a stirred solution of 2-exo-bromo-3-endo-[(tetrahydro-2H-pyran-2-yl)oxy]bicyclo[3.2.0]heptan-6-one (100.8 g) in acetone (500 ml) at 0°. After 2 h at 5° the mixture was stirred at 20° for 18 h and then filtered. Evaporation of the filtrate gave an oil which was taken into ether (350 ml), filtered and washed (water, 2 × 100 ml). The ethereal solution was dried ($MgSO_4$), filtered and evaporated to give the *title compound* as a solid. Purification from PE gave material (85.5 g) of m.p. 86—88°.

Intermediate 13

(a) *1-Bromomethyl-4-phenylsulphonyl benzene*

A solution of p-tolyl phenylsulphone (20 g) in refluxing $CCl_4$ (400 ml) containing dibenzoyl peroxide (0.4 g) was treated with N-bromosuccinimide (15.3 g) in portions rapidly. The mixture was heated under reflux for 30 min., filtered whilst hot and the filtrate allowed to cool. Evaporation gave the *title compound* (26.8 g), m.p. 103—104°.

(b) *N,N-Dimethyl, 4-bromomethylbenzenesulphonamide,*
m.p. 94—96° was prepared by a similar procedure from N,N-dimethyl-p-toluenesulphonamide.

Intermediate 14

*4-(1,3-Dioxolan-2-yl)-2-phenylthiophene*

A solution of 5-bromo-3-thiophenecarboxaldehyde (32.5 g) in benzene (500 ml) was treated with PTSA (0.323 g) and ethylene glycol (21.1 g), and the mixture heated under reflux in a Dean and Stark apparatus until the theoretical volume of water had been removed. After cooling the mixture was washed with water, (2 ×) then brine, dried ($MgSO_4$), filtered and concentrated, and the residue distilled (b.p. 96—100° at 0.4 mm) to give the *title compound* as an oil (24 g).

| | | |
|---|---|---|
| Analysis Found: | C, 35.8; | H, 3.0; |
| $C_7H_7BrO_2S$ requires: | C, 35.7; | H, 3.0%. |

Intermediate 15

(a) *5-Phenyl-3-thiophenecarboxaldehyde*

A solution of phenylmagnesium chloride in THF (82.94 ml, 2.39 M) was added to a stirred solution of $ZnBr_2$ (44.6 g) in dry THF (350 ml) under nitrogen. The mixture was stirred at room temperature for 15 min.

Dibal (9.91 ml, 1 M) in hexane solution was added dropwise to a stirred mixture of triphenylphosphine (10.39 g) and nickel acetoacetonate (2.55 g) in dry THF (160 ml) under nitrogen. A solution of Intermediate 14 (23.3 g) in dry THF (150 ml) was added after 10 min. The solution contaning the organozinc reagent was then added dropwise and the mixture was stirred for 1 h.

2N Hydrochloric acid (400 ml) was added at 0° and the mixture was stirred at room temperature for 0.5 h. The two layers were separated and the aqueous layer was extracted with ether (2 × 400 ml), washed with $NaHCO_3$ solution and brine and then dried ($MgSO_4$). Solvent removal *in vacuo* gave a solid (32.8 g) which was chromatographed (A) to give the *title compound* (13.35 g), m.p. 64—65° (from PE (b.p. 60—80°)).

The following compounds were prepared by a similar procedure:

(b) *4-(4-Methoxyphenyl)-2-thiophenecarboxaldehyde*, m.p. 75—76.5° (from PE (b.p. 60—80°)—EA, 2:1), from 4-bromo-2-(1,3-dioxolan-2-yl)thiophene and p-methoxyphenyl zinc bromide [from 4-bromoanisole, zinc bromide]

(c) *4-Phenylmethyl)-2-thiophene carboxaldehyde,* from 4-bromo-2-(1,3-dioxolan-2-yl)thiophene and a solution of activated zinc dust and benzyl bromide in dry THF. Purification by chromatography (B)

| | | |
|---|---|---|
| Analysis Found: | C, 71.3; | H, 5.0 |
| $C_{12}H_{10}OS$ requires: | C, 71.3; | H, 5.0%. |

Intermediate 16

*4-(4-Methoxyphenyl)-2-thiophenemethanol*

Intermediate 15(b) (9.85 g) in THF (40 ml) and absolute ethanol (160 ml) was stirred with $NaBH_4$ (1.9 g) at room temperature for 1H. Saturated aqueous $KH_2PO_4$ (60 ml) was added and the mixture evaporated *in vacuo*. Extraction of the residue with $CH_2Cl_2$ (3 × 50 ml), drying ($MgSO_4$) and evaporation gave a solid. Crystallisation from EA gave the *title compound* (7.49 g), m.p. 136.5—138°.

Intermediate 17

(a) *5-Phenyl-3-thiophenemethanol*

A stirred solution of Intermediate 15 (12 g) in methanol (120 ml) was treated with $NaBH_4$ (1.82 g) at room temperature for 15 min. The mixture was cooled to 0° and treated with $NH_4Cl$ solution (200 ml), followed by water (200 ml) and ether (400 ml). The ether extract was separated and the aqueous phase further extracted with ether (400 ml), washed with brine, dried ($MgSO_4$), filtered and evaporated to afford the *title compound* as a solid (11.5 g), m.p. 92—93°.

(b) *4-(Phenylmethyl)-2-thiophene methanol,* m.p. 33—34° was prepared by a similar procedure from Intermediate 15(c).

Intermediate 18

*2-(1-Cyclohexenyl)thiophene*

n-Butyllithium (40.7 ml, 1.5 M) was added dropwise to a stirred solution of thiophene (5 g) in dry ether (50 ml) and the mixture heated under reflux for 30 min. After cooling to −78°, cyclohexanone (6.21 ml) in dry ether (30 ml) was added dropwise and the temperature allowed to rise to ambient. After 1 h 2 N hydrochloric acid (80 ml) was added and stirring continued for 16 h. The organic layer was separated and the aqueous layer extracted with ether (2 × 40 ml). The combined extracts were washed with water, dried ($MgSO_4$), filtered and evaporated to give an oil, which was dissolved in benzene (50 ml) and heated under reflux in the presence of PTSA for 0.75 h. The cooled solution was washed twice with 8% $NaHCO_3$ solution

and the aqueous solution extracted with ether (2 × 40 ml), dried ($MgSO_4$) filtered and evaporated to give the *title compound* as an oil (9.83 g). TLC (F) $R_f$ 0.73.

Intermediate 19
*2-Cyclohexylthiophene*

A solution of Intermediate 18 (9.8 g) in absolute alcohol (75 ml) was hydrogenated over prereduced 10% palladium oxide on charcoal (2 g). The mixture was filtered ('Hyflo') and the filtrate evaporated. Distillation of the residue gave the *title compound* (6.6 g) of b.p. 70—80/0.2 mm.

Intermediate 20
*5-Cyclohexyl-2-thiophenemethanol*

n-Butyllithium (25.3 ml, 1.5 M) was added dropwise to a stirred solution of Intermediate 19 (6 g) in dry THF (75 ml). After 15 min the solution was cooled to 0° and paraformaldehyde (3.25 g) was added. After a further 20 min at room temperature saturated $NH_4Cl$ solution (30 ml) was added, the organic layer separated and the aqueous solution extracted with ether (2 × 50 ml), dried ($MgSO_4$), filtered and evaporated, and the residue chromatographed (C). The *title compound* was obtained as a solid (5.36 g), m.p. 31—31.5°.

Intermediate 21
(a) *4-Phenyl-2-thiophenemethanol*

A stirred suspension of 4-phenyl-2-thiophenecarboxaldehyde (4.32 g) in absolute ethanol (85 ml) was cooled in an ice-bath and treated with $NaBH_4$ (1.06 g). After 20 min the mixture was allowed to attain ambient temperature when stirring was continued for 6 h. Saturated aqueous $NH_4Cl$ (30 ml) was then carefully added to the vigorously stirred mixture, and the resulting suspension extracted with ether (2 × 200 ml). The combined extracts were dried ($Na_2SO_4/K_2CO_3$), filtered and evaporated to give the *title compound* (4.2 g) as crystals, m.p. 112—113°.

(b) *4-Bromo-2-thiophene methanol* was similarly prepared from 4-bromo-2-thiophene carboxaldehyde.

| | | |
|---|---|---|
| Analysis Found: | C, 31.1; | H, 2.6 |
| $C_5H_5BrOS$ requires: | C, 30.8; | H, 2.6%. |

Intermediate 22
*4-Methyl-2-thiophenemethanol*

A solution of 4-methyl-2-thiophene carboxylic acid (6 g) in dry ether (50 ml) was added to a stirred suspension of $LiAlH_4$ (2 g) in dry ether (100 ml) and the mixture kept at room temperature for 2 h and 30° for 1 h. Wet THF (50 ml) was cautiously added followed by 1 N hydrochloric acid (150 ml). The layers were separated and the aqueous solution extracted with ether (100 ml), washed with $Na_2CO_3$ solution (2 ×), water (2 ×), brine and then dried ($MgSO_4$). Evaporation gave a liquid which was distilled (b.p. 90°/1 mm) to give the *title compound* (4.25 g).

Intermediate 23
(a) *2-(Bromomethyl)-4-phenylthiophene*

A cooled, stirred suspension of Intermediate 21a) (3.86 g) in dry $CH_2Cl_2$ (60 ml) was treated dropwise with a solution of $PBr_3$ (1.27 ml) in dry $CH_2Cl_2$ (20 ml), and stirring continued for 30 min. The mixture was treated with 8% aqueous $NaHCO_3$ (100 ml), stirred for 20 min., extracted with ether (1 × 150 ml, 1 × 50 ml), and the extracts dried ($MgSO_4$), filtered and evaporated to give the *title compound* (51.01 g) as a solid, m.p. 87—88.5°.

The following compounds were prepared by a similar procedure:

(b) *2-Bromomethyl-4-(phenylmethyl)thiophene,* from Intermediate 17b); TLC (M) $R_f$ 0.58.

(c) *2-Bromomethyl-5-cyclohexylthiophene,* from Intermediate 20; TLC (M) $R_f$ 0.72.

(d) *4-Bromo-2-(bromomethyl)thiophene,* from Intermediate 21b),

| | | |
|---|---|---|
| Analysis Found: | C, 23.5; | H, 1.6. |
| $C_5H_4Br_2S$ requires: | C, 23.5; | H, 1.6%. |

(e) *2-(Bromomethyl)-4-(4-methoxyphenyl)thiophene,* from Intermediate 16;

| | | |
|---|---|---|
| Analysis Found: | C, 50.6; | H, 4.1. |
| $C_{12}H_{11}BrOS$ requires: | C, 50.9; | H, 3.9%. |

(f) *4-(Bromomethyl)-2-phenylthiophene,* from Intermediate 17; TLC (J) Rf 0.58.

(g) *2-Bromomethyl-4-methylthiophene,* from Intermediate 22; TLC (O) Rf 0.55.

Intermediate 24
*2-(Bromomethyl)-5-phenylthiophene*
To a boiling solution of 2-methyl-5-phenylthiophene (2 g) in dry $CCl_4$ (100 ml) was rapidly added N-bromosuccinimide (1.94 g) and dibenzoylperoxide (0.15 g). After heating under reflux for 30 min., the mixture was cooled and filtered. Evaporation *in vacuo* gave the *title compound* as an oil (1.5 g).

| | | |
|---|---|---|
| Analysis Found: | C, 52.0; | H, 3.4; |
| $C_{11}H_9BrS$ requires: | C, 52.2; | H, 3.6%. |

Intermediate 25
*(±)-5-endo-Hydroxy-7-anti-(4-morpholinyl)bicyclo[2.2.1]heptan-2-one, hydrochloride*
To a stirred solution of Intermediate 12 (96.4 g) in methanol (600 ml) was added an ethereal solution of HCl (240 ml) and the mixture stirred at 20° for 2.5 h (pH 1.5—2). Filtration followed by evaporation of the filtrate gave an oil which solidified on trituration with EA (2 × 200 ml). Coloured impurities were removed by extraction with boiling isopropanol to leave the *title compound* as a solid (70.6 g), m.p. 181—182°.

Intermediate 26
(a) *(±)-5-endo-(4-Bromophenylmethoxy)-7-anti-(4-morpholinyl)bicyclo[2.2.1]heptan-2-one*
Aqueous NaOH solution (10N; 200 ml) was added to a solution of the free base of Intermediate 25 (21.1 g), benzyltriethylammonium chloride (4 g) and 4-bromobenzyl bromide (27.5 g) in $CH_2Cl_2$ (400 ml) and the mixture stirred vigorously for 4 h. A further portion of 4-bromobenzylbromide (9 g) was then added and stirring continued for 68 h. Water (200 ml) was added and the layers separated. The aqueous layer was extracted with EA (2 × 75 ml), washed with water, dried ($MgSO_4$) and evaporated to give an oil (48 g) which solidified on standing. Excess alkylating agent was removed by trituration with PE (b.p. 60—80°) and crystallisation from EA—PE (b.p. 60—80°) then gave the *title compound* (34.1 g) as a solid, m.p. 130—131°.
The following compounds were prepared by a similar procedure:

(b) *(±) - 7 - anti - (4 - Morpholinyl) - 5 - endo - [4 - phenylsulphonyl(phenylmethoxy)]bicyclo[2.2.1]-heptan - 2 - one,* m.p. 133—135°, from Intermediates 25 and 13a). Purification by chromatography twice (D), then (E)

(c) *7 - anti - (4 - Morpholinyl) - 5 - endo - [(5 - phenylthien - 2 - yl)methoxy]bicyclo[2.2.1]heptan - 2 - one,* m.p. 129—130°, from Intermediates 25 and 24. Purification by chromatography (F).

(d) *5 - endo - [(5 - Cyclohexylthien - 2 - yl)methoxy] - 7 - anti - (4 - morpholinyl)bicyclo[2.2.1]heptan - 2 - one,* m.p. 56—57°, from Intermediates 25 and 23c). Purification by chromatography (G)

Intermediate 27
*(±)-8-anti-(4-Morpholinyl)-6-endo-[4-phenylsulphonyl(phenylmethoxy)]cyclopentyl]-2-oxabicyclo[3.2.1]octan-3-one*
To a stirred solution of Intermediate 26b) (2.12 g) in $CH_2Cl_2$ (22 ml) at 0° was added peracetic acid (3.14 ml, 6.12 M) and the resulting solution then stirred at ambient temperature for 18 h. Excess $Na_2SO_3$ solution was added to the cooled solution and stirring continued for 1 h. After evaporation *in vacuo* the residue was suspended in $NaHCO_3$ solution (50 ml), extracted with EA (3 × 70 ml), dried ($MgSO_4$), filtered and concentrated to give the *title compound* as a solid (1.16 g), m.p. 170—171° (from EA-acetone-PE).

Intermediate 28
(a) *(±)-6-endo-(4-Bromophenylmethoxy)-8-anti-(4-morpholinyl)-2-oxabicyclo[3.2.1]octan-3-one*
Intermediate 26a) (13.2 g) in acetic acid (110 ml) and water (55 ml) containing $CH_3COONa.3H_2O$ (23.7 g) was cooled (*ca.* 5—10°) and stirred during the dropwise addition of peracetic acid (6.1 M; 28.5 ml). The resulting solution was stirred at 20° for 48 h when 10% $Na_2SO_3$ solution (200 ml), was added, maintaining the temperature of the mixture at 10—15°. After 1.5 h solvents were removed *in vacuo* at 35°, the residue taken into water (150 ml) and basified to pH 9 with $Na_2CO_3$ solution. Extraction with EA (3 × 200 ml) followed by drying and evaporation gave a solid which crystallised from EA to give the *title compound* (5.49 g), m.p. 154—156°.
The following compounds were prepared by a similar procedure:

(b) *8 - anti - (4 - Morpholinyl) - 6 - endo[(5 - phenylthien - 2 - yl)methoxy] - 2 - oxabicyclo[3.2.1]octan - 3 - one,* m.p. 135—137°, from Intermediate 26c). Purification by chromatography (H) through to (I).

(c) *6 - endo - [(5 - Cyclohexylthien - 2 - yl)methoxy] - 8 - anti(4 - morpholinyl) - 2 - oxabicyclo[3.2.1]octan - 3 - one,* from Intermediate 26d). TLC ($SiO_2$) Ether $R_f$ 0.38. Purification by chromatography (J)

Intermediate 29
*(±)-8-anti-(4-Morpholinyl)-6-endo-[[[4-(phenylmethyl)phenyl]-4-yl]methoxy]-2-oxabicyclo[3.2.1]octan-3-one*

To a stirred suspension of activated Zn dust (13.08 g) in THF (40 ml) was added dropwise over 0.5 h a solution of benzyl bromide (11.9 ml) in THF (40 ml), the internal temperature being maintained at 10°. The mixture was then stirred at 10—15° for 1 h.

Nickel acetylacetonate (0.774 g) and triphenylphosphine (3.14 g) were dissolved in THF (15 ml) and stirred at 20° under nitrogen during the dropwise addition of Dibal (1 M in hexane, 3 ml). After 5 min a solution of Intermediate 28a) (2.77 g) in THF (35 ml) was added, followed after a further 5 min by the solution of benzylzinc bromide described above. The mixture was stirred at 29° for 19 h and then poured into $NH_4Cl$ solution (250 ml) and EA (150 ml). 2 N hydrochloric acid was added to pH 6 and the layers were separated. The aqueous solution was extracted with EA (100 ml), dried and concentrated. Purification by chromatography (K). Crystallisation from EA—PE gave the *title compound* (2.3 g), m.p. 119—121°.

Intermediate 30
(a) *(±)-6-endo-[[4'-Methoxy(1,1'-phenyl)-4-yl]methoxy]-8-anti-(4-morpholinyl)-2-oxabicyclo-[3.2.1]octan-3-one*

4-Bromoanisole (7.48 g) in ether (30 ml) was added to Mg turnings (1.06 g) in THF (40 ml) containing a small iodine crystal. After 0.5 h an exothermic reaction took place which was moderated by water bath cooling. The resultant mixture was stirred at 20° for a further 0.5 h and then added, under nitrogen, to a stirred, cooled (5°) solution of anhydrous $ZnBr_2$ (9.0 g) in THF (40 ml) and stirred for 1 h.

Dibal (1 M in hexane; 5.72 ml) was added dropwise to a suspension of bis(triphenylphosphine)-palladium chloride (2.0 g) in THF (10 ml). After stirring for 5 min a solution of Intermediate 28a) (2.38 g) in THF (35 ml) was added followed after a further 5 min by the solution of 4-methoxyphenylzinc bromide described above. The resultant mixture was stirred under nitrogen at 20° for 18 h. The solvent was then removed *in vacuo*, $NH_4Cl$ solution (150 ml) added and the mixture extracted with EA (3 × 100 ml). Evaporation of the dried extracts gave an oil which was chromatographed (K) to give the *title compound* as a solid. Crystallisation from EA—PE (b.p. 60—80°) gave material (1.58 g) of m.p. 123—125°.

(b)*(±) - 8- anti - (4 - Morpholinyl) - 6- endo - [[(1,1':4,1'' - terphenyl) - 4 - yl]methoxy] - 2 - oxabicyclo-[3.2.1]octan - 3 - one,* m.p. 196—198° was similarly prepared from Intermediate 28a) and biphenylzinc chloride.

(c) *(±) - 6 - endo - [4 - (Thien - 2 - yl)phenylmethoxy] - 8 - anti - (4 - morpholinyl) - 2 - oxabicyclo[3.2.1]-octan - 3 - one,* m.p. 216—217° was similarly prepared from Intermediate 28a) and a solution of anhydrous $ZnBr_2$ and the Grignard reagent fro 2-bromothiophene and Mg in dry THF. Purification by chromatography, EA—PE (b.p. 60—80°) (1:2, 1:1 and 2:1 successively).

Intermediate 31
*(3aα,4α,5β26aα)-(±)-Hexahydro-5-hydroxy-4-(4-thiomorpholinyl)-2H-cyclopenta(b)furan-2-one, S-dioxide, hydrochloride*

A solution of the product of Preparation 30 (British Patent Specification 2028805A) (10 g) in ethanol (60 ml) and water (40 ml) containing concentrated hydrochloric acid (40 ml) was hydrogenated over prereduced 10% palladium oxide on charcoal (5 g, 50% dispersion in water) in ethanol (40 ml). The mixture was filtered and the filtrate evaporated *in vacuo* to give the *title compound* as a solid (8.55 g), m.p. above 230° (dec.) (from water-ethanol).

Intermediate 32
*(3aα,4α,5β,6aα)-(±)-Hexahydro-5-[(tetrahydro-2H-pyran-2-yl)oxy]-4-(4-thiomorpholinyl)-2H-cyclopenta(b)-furan-2-one, S-dioxide*

Dihydropyran (3.1 ml) was added to a stirred solution of the free base of Intermediate 31 (1.56 g) and PTSA (1.17 g) in dry DMF (30 ml) at −10°. The mixture was allowed to reach ambient temperature and stirring continued for 18 h, whereupon it was poured into saturated aqueous $NaHCO_3$ solution (50 ml), extracted with EA (4 × 100 ml), washed with water, dried ($MgSO_4$), filtered and concentrated. The residue was chromatographed (L) to give the *title compound* as a viscous oil (1.89 g). IR ($CHBr_3$) 1762 $cm^{-1}$.

Intermediate 33
(a) *(1α,2β3α5α)-(±)-3-Hydroxy-5-[[4'-methoxy(1,1'-biphenyl)-4-yl]methoxy]-2-(4-morpholinyl)-cyclopentane acetaldehyde*

A solution of Intermediate 30a) (1.6 g) in $CH_2Cl_2$ (25 ml) at −70° under dry nitrogen was stirred during the addition of Dibal (1 M in hexane, 8.7 ml). After 1.5 h at −70°, methanol (25 ml) was carefully added and the mixture was then allowed to rise to ambient temperature whereupon stirring was continued for 18 h.

The mixture was filtered through 'Hyflo' and the filtrate evaporated to give the *title compound* as a foam (1.68 g). IR($CHBr_3$) 3580/3560(br), 1715, 1240, 1040 $cm^{-1}$.

The following compounds were prepared by a similar procedure:

(b) *(1α,2β,3α,5α) - (±) - 3 - Hydroxy - 2 - (4 - morpholinyl) - 5 - [[(1,1':4,1'' - terphenyl) - 4 - yl]methoxy]-cyclopentane acetaldehyde,* m.p. 154—156°C from Intermediate 30b).

(c) *(1α,2β,3α,5α) - (±) - 3 - Hydroxy - 2 - (4 - morpholinyl) - 5 - [4 - (phenylmethyl)phenyl]methoxy]cyclopentane acetaldehyde,* from Intermediate 29, IR ($CHBr_3$) 3590, 1715 $cm^{-1}$.

(d) *(1α,2β,3α,5α) - (±) - 3 - Hydroxy - 2 - (4 - morpholinyl) - 5 - [4 - (thien - 2 - yl)phenylmethoxy]cyclopentane acetaldehyde,* from Intermediate 30c), TLC (U) Rf 0.3.

(e) *(1α,2β,3α,5α) - (±) - 3 - Hydroxy - 2 - (4 - morpholinyl) - 5 - [4 - phenylsulphonyl(phenylmethoxy)]-cyclopentane acetaldehyde,* from Intermediate 27, IR ($CHBr_3$) 1705 $cm^{-1}$.

(f) *(3aα,4α,5β,6aα) - (±) - Hexahydro - 5 - [(tetrahydro - 2H - pyran - 2 - yl)oxy] - 4 - (4 - thiomorpholinyl) - 2H - cyclopenta(b) - furan - 2 - ol, S - dioxide,* from Intermediate 32

| | | | |
|---|---|---|---|
| Analysis Found: | C, 53.2; | H, 7.6; | N, 3.5. |
| $C_{16}H_{27}NO_6S$ requires: | C, 53.2; | H, 7.5; | N, 3.9%. |

Intermediate 34

(a) *(1α,2β,3α,5α)-(±)-3-Hydroxy-2-(4-morpholinyl)-5-[(5-phenylthien-2-yl)methoxy]cyclopentane acetaldehyde*

A stirred solution of Intermediate 28b (0.5 g) in dry $CH_2Cl_2$ (20 ml) at −70° under nitrogen was treated dropwise with Dibal (1M in hexane, 2.5 ml). After 1h at −70°, methanol (20 ml) was added and the temperature of the mixture allowed to rise to ambient over 3h. The mixture was filtered through 'Hyflo', concentrated and the residue taken up into $CH_2Cl_2$ (100 ml). After drying ($MgSO_4$), filtration and concentration gave the *title compound* as a foam (0.4 g). IR ($CHBr_3$) 1715 $cm^{-1}$.

(b) *[1α,2β,3α,5α] - (±) - 5 - [(5 - Cyclohexylthien - 2 - yl)methoxy] - 3 - hydroxy - 2 - (4 - morpholinyl)cyclopentane acetaldehyde,* was prepared by a similar procedure from Intermediate 28c), IR ($CHBr_3$) 3580—3530 (broad), 1710 $cm^{-1}$.

Intermediate 35

*[1α(Z),2β,3α,5α]-(±)-Methyl 7-[3-Acetoxy-5-[4-acetylamino(phenylmethoxy)]-2-(4-morpholinyl)cyclopentyl]-5-heptenoate*

Intermediate 10 (0.7 g) in acetic anhydride (10 ml) and pyridine (15 ml) was stood at room temperature for 18h. After evaporation *in vacuo* the residue was treated with 8% aqueous $NaHCO_3$ solution (50 ml), extracted with ether (3 × 30 ml), dried ($MgSO_4$), filtered and concentrated to give the *title compound* as an oil (0.62 g). IR ($CHBr_3$) 3430, 1730, 1690, 1510 $cm^{-1}$.

Intermediate 36

(a) *[1α(Z),2β,3α,5α]-(±)-Methyl 7-[2-(4-Morpholinyl)-5-[[4-phenylmethyl)thien-2-yl]methoxy-3-[(tetrahydro-2H-pyran-2-yl)oxy]cyclopentyl]-5-heptenoate*

Sodium hydride (1.34 g, 50% in oil) was added to a stirred solution of Intermediates 9 (3.83 g) and 23b) (7.75 g) in dry DMF (20 ml) at 0° under nitrogen. The mixture was stirred at 0° for 15 min and then at room temperature for 1h, whereupon $NH_4Cl$ solution (100 ml) was added and the pH adjusted to 6.5 with $KH_2PO_4$ solution. The mixture was extracted with ether (3 × 100 ml), washed with water (2×) and brine and then dried ($MgSO_4$). Evaporation *in vacuo* gave a residue which was purified by chromatography (M) to give the *title compound* as an oil (2.1 g).

| | | | |
|---|---|---|---|
| Analysis Found: | C, 67.9; | H, 8.1; | N, 2.2. |
| $C_{34}H_{47}NO_6S$ requires: | C, 68.3; | H, 7.9; | N, 2.4%. |

The following compounds were prepared by a similar procedure:

(b) *[1α(Z),2β,3α,5α] - (±) - Methyl 7 - [5 - [(4 - Bromothien - 2 - yl)methoxy] - 2 - (4 - morpholinyl) - 3 - [(tetrahydro - 2H - pyran - 2 - yl)oxy]cyclopentyl] - 5 - heptenoate* from Intermediates 9 and 23a). Purification by chromatography (N)

| | | | |
|---|---|---|---|
| Analysis Found: | C, 55.5, | H, 6.7; | N, 2.1 |
| $C_{27}H_{40}BrNO_6S$ requires: | C, 55.3; | H, 6.9 | N, 2.4%. |

(c) *[1α(Z),2β,3α,5α] - (±) - Methyl 7 - [5 - [[4 - (4 - Methoxyphenyl)thien - 2 - yl]methoxy - 2 - (4 - morpholinyl) - 3 - [(tetrahydro - 2H - pyran - 2 - yl)oxy]cyclopentyl] - 5 - heptenoate* from Intermediates 9 and 23e). Purification by chromatography (O).

| | | | |
|---|---|---|---|
| Analysis Found: | C, 66.6; | H, 7.8; | N, 2.4 |
| $C_{34}H_{47}NO_7S$ requires: | C, 66.5 | H, 7.71; | N, 2.3%. |

Intermediate 37
*[1α(Z),2β,3α,5α]-(±)-Methyl 7-[5-Hydroxy-3-[(tetrahydro-2H-pyran-2-yl)oxy]-2-(4-thiomorpholinyl)-cyclopentyl]-5-heptanoate, S-oxide*

To a stirred solution of potassium t-butoxide (1.12 g) in dry THF (10 ml) under dry nitrogen was added (4-carboxybutyl)triphenylphosphonium bromide (2.21 g) and the mixture stirred at 22° for 15 min., whereupon a solution of Intermediate 33f) (0.9 g) in THF (5 ml) was added and stirring continued for a further 30 min. Water (50 ml) was added and the mixture extracted with EA (3 × 25 ml). The aqueous phase was adjusted to pH 6.5 with $KH_2PO_4$ solution and then extracted with EA (3 × 30 ml), washed with brine, dried ($MgSO_4$) and treated with ethereal diazomethane. Concentration gave an oil which was chromatographed (L) to give the *title compound* (0.63 g).

| | | | |
|---|---|---|---|
| Analysis Found: | C, 57.8; | H, 8.3; | N, 2.9 |
| $C_{22}H_{37}NO_7S$ requires: | C, 57.5; | H, 8.1; | N, 3.1%. |

Intermediate 38
*[1α(Z),2β,3α,5α]-(±)-Methyl 7-[3-Acetoxy-5-[4-N,N-dimethylaminosulphonyl(phenylmethoxv)]-2-(4-morpholinyl)cyclopentyl]-5-heptenoate*

Intermediates 9 (2.5 g) and 13b (5.08 g) were converted into the hydroxy analogue of the *title compound* by the method of Example 1. The residue was treated with acetic anhydride (0.9 ml) in dry pyridine (10 ml) at room temperature for 18h. The mixture was diluted with ether, washed with $NaHCO_3$ solution and brine, dried ($MgSO_4$) and concentrated. Purification of the residue by chromatography (J) gave the *title compound* as an oil (0.8 g).

IR (Neat) 1730, 1340, 1160 $cm^{-1}$.

Intermediate 39
*4-Bromomethyl-4'-chloro-1,1'-biphenyl*

4'-Chloro(1,1'-biphenyl)-4-methanol (5.8 g) was converted into the *title compound* (6.8 g), m.p. 64—66° by the method for the preparation of Intermediate 3.

Intermediate 40
*[1α(Z),2β,3α,5α]-(±)-Methyl 7-[5-[[4'-Chloro(1,1'-biphenyl)-4-yl]methoxy]-2-(4-morpholinyl)-3-[(tetrahydro-2H-pyran-2-yl)oxy]cyclopentyl]-5-heptenoate*

To a stirred solution of Intermediate 9 (2.6 g) and NaH (75% dispersion in oil, 0.6 g) in dry DMF (20 ml) was added after 5 min. a solution of Intermediate 39 (5.3 g) in DMF (10 ml). The mixture was stirred for 5h at room temperature, then poured into $NH_4Cl$ solution and extracted with ether. The dried ($MgSO_4$) extracts were evaporated and the residue chromatographed eluting successively with (1:4; 1:2; 1:1; 2:1) EA—PE (b.p. 60—80°) and 4:1 EA-methanol to give the *title compound* as an oil (1.8 g).

IR (Neat) 1732, 1090 $cm^{-1}$.

## Example 1

(a) *[1α(Z),2β,3α,5α]-(±)-Methyl 7-[5-[[4'-methyl(1,1'-biphenyl)-4-yl]methoxy]-3-hydroxy-2-(4-morpholinyl)cyclopentyl]-5-heptenoate*

A solution of Intermediates 9 (0.43 g) and 3 (0.68 g) in dry DMF (6 ml) was stirred at 0° under nitrogen during the addition of NaH (0.08 g, 80% dispersion in oil). After 4h at 20°, the mixture was carefully poured into saturated aqueous $NH_4Cl$ (70 ml) and extracted with ether (3 × 40 ml). The combined extracts were dried ($MgSO_4$), filtered and evaporated, and the residue then stirred with 5% methanolic sulphuric acid (20 ml) at 20° for 2h. The mixture was poured into 8% $NaHCO_3$ solution (100 ml), extracted with ether

(3 × 50 ml), dried ($MgSO_4$), evaporated and the residue purified by chromatography (P). The *title compound* was crystallised from ether-PE as needles (0.14 g), m.p. 75—77°.

The following compounds were prepared by a similar procedure:

(b) *[1α(Z),2β,3α,5α] - (±) - Methyl 7 - [3 - Hydroxy - 5 - [4 - methylthio(phenylmethoxy)] - 2 - (4 - morpholinyl)cyclopentyl] - 5 - heptenoate* from Intermediate 11 and 1-(bromomethyl)-4-(methylthio)benzene

| | | | |
|---|---|---|---|
| Analysis Found: | C, 65.1; | H, 8.0; | N, 2.9; |
| $C_{25}H_{37}NO_5S$ requires: | C, 64.8; | H, 8.0; | N, 3.0% |

(c) *[1α(Z),2β,3α,5α] - (±) - Methyl 7 - [3 - Hydroxy - 2 - (4 - morpholinyl) - 5 - [4 - (2 - phenylethyl)phenylmethoxy]cyclopentyl] - 5 - heptenoate,* from Intermediates 9 and 1, IR (Neat) 3460, 1740 $cm^{-1}$. Purification by chromatography (Q).

(d) *[1α(Z),2β,3α,5α] - (±) - Methyl 7 - [3 - Hydroxy - 5 - [[4' - methoxy(1,1' - biphenyl) - 4 - yl]methoxy] - 2 - (1 - piperidinyl)cyclopentyl] - 5 - heptenoate,* IR (Neat) 1735 $cm^{-1}$, from Intermediate 41 and 4-bromomethyl-4'-methoxy(1,1'-biphenyl). Purification by chromatography (R).

(e) *[1α(Z),2β,3α,5α] - (±) - Methyl 7 - [3 - Hydroxy - 5 - [4 - (phenylmethyl)phenylmethoxy] - 2 - (1 - piperidinyl)cyclopentyl] - 5 - heptenoate,* IR (Neat) 3440, 1735 $cm^{-1}$, from Intermediate 41 and 1-bromomethyl-4-(phenylmethyl)benzene. Purification by chromatography (R).

(f) *[1α(Z),2β,3α,5α] - (±) - Methyl 7 - [3 - Hydroxy - 5 - [[4' - methyl(1,1' - biphenyl) - 4 - yl]methoxy] - 2 - (1 - piperidinyl)cyclopentyl] - 5 - heptenoate,* from Intermediate 41 and Intermediate 3, purification by chromatography (S). IR ($CHBr_3$) 3600/3500, 1722 $cm^{-1}$. TLC (S) $R_f$ 0.28.

(g) *[1α(Z),2β,3α,5α] - (±) - Methyl 7 - [3 - Hydroxy - 5 - [[4' - methoxy(1,1' - biphenyl) - 4 - yl]methoxy] - 2 - (4 - thiomorpholinyl)cyclopentyl] - 5 - heptenoate, S-dioxide* from Intermediate 37 and 4-bromomethyl-4'-methoxy(1,1'-biphenyl). Purification by chromatography (L). IR ($CHBr_3$) 3580/3500, 1723 $cm^{-1}$. TLC (I) $R_f$ 0.36.

(h) *[1α(Z),2β,3α,5α] - (±) - Methyl 7 - [3 - Hydroxy - 5 - [4 - (phenylmethyl)phenylmethoxy] - 2 - (4 - thiomorpholinyl)cyclopentyl] - 5 - heptenoate, S-dioxide,* from Intermediate 37 (1g) and 1-bromomethyl-4-(phenylmethyl)benzene (1.76 g). Purification by chromatography (J) increasing to (Q). IR ($CHBr_3$) 3580—3480 (br.), 1730 $cm^{-1}$. TLC (Q) $R_f$ 0.42.

(i) *[1α(Z),2β,3α,5α] - (±) - Methyl 7 - [3 - Hydroxy - 5 - [[4' - methyl(1,1' - biphenyl) - 4 - yl]methoxy] - 2 - (4 - thiomorpholinyl)cyclopentyl] - 5 - heptenoate, S-dioxide,* from Intermediates 37 and 3. Purification by chromatography (L). IR (Neat) 3520 (br.), 1740, 1302, 1123 $cm^{-1}$. TLC (I). $R_f$ 0.61.

(j) *[1α(Z),2β,3α,5α] - (±) - Methyl 7 - [3 - Hydroxy - 2 - (4 - morpholinyl) - 5 - (2 - thienylmethoxy)cyclopentyl] - 5 - heptenoate,* from Intermediate q and 2-bromomethyl thiophene, IR (Neat) 3410, 1730 $cm^{-1}$. TLC ($SiO_2$) 95:5 chloroform-methanol $R_f$ 0.4. Purification by chromatography (T).

(k) *[1α(Z),2β,3α,5α] - (±) - Methyl 7 - [3 - Hydroxy - 2 - (4 - morpholinyl) - 5 - [(2 - phenylthien - 4 - yl)methoxy]cyclopentyl] - 5 - heptenoate* from Intermediates 9 and 23f). IR ($CHBr_3$) 3590, 1628 $cm^{-1}$. Purification by chromatography (I).

(l) *[1α(Z),2β,3α,5α] - (±) - Methyl 7 - [3 - Hydroxy - 2 - (4 - morpholinyl) - 5 - (3 - thienylmethoxy)cyclopentyl] - 5 - heptenoate,* from Intermediate 9 and 3-bromomethylthiophene. Purification by chromatography using 1—4% methanol in ether as eluent. IR ($CHBr_3$) 3500, 1735 $cm^{-1}$. TLC (Q) $R_f$ 0.34.

(m) *[1α(Z),2β,3α,5α] - (±) - Methyl 7 - [3 - Hydroxy - 5 - [(4 - methylthien - 2 - yl)methoxy] - 2 - (4 - morpholinyl)cyclopentyl] - 5 - heptenoate,* from Intermediates 9 and 23 g). Purification by chromatography (L). IR ($CHBr_3$) 3580 (Broad), 1728 $cm^{-1}$.

(n) *[1α(Z),2β,3α,5α] - (±) - 7 - [3 - Hydroxy - 2 - (4 - morpholinyl) - 5 - [(5 - phenylfuran - 2 - yl)methoxy]cyclopentyl] - 5 - heptenoic acid,* from Intermediate 9 and 2-(bromomethyl)-5-phenylfuran, IR($CHBr_3$) 3500, 1720, 1700, 1018, 788 $cm^{-1}$. TLC ($SiO_2$) (I) $R_f$ 0.18. Purification by chromatography (I).

(o) *[1α(Z),2β,3α,5α] - (±) - Methyl 7 - [3 - Hydroxy - 2 - (4 - morpholinyl) - 5 - [(4 - phenylthien - 2 - yl)methoxy)cyclopentyl] - 5 - heptenoate*, from Intermediates 9 and 23a), m.p. 78—80°. Purification by chromatography (K) followed by (U).

Example 2

(a)*[1α(Z),2β,3α,5α]-(±)-7-[3-Hydroxy-5-[[4'-methyl(1,1'-biphenyl)-4-yl]methoxy-2-(4-morpholinyl)-cyclopentyl]-5-heptenoic acid*

A suspension of the product of Example 1a) (0.44 g) in methanol (2 ml) and water (4 ml) containing KOH (0.35 g) was stirred at room temperature for 7h. The methanol was removed and the residue further diluted with water (75 ml), washed with ether (75 ml) and then carefully acidified to pH 6 with 2N hydrochloric acid. Extraction with ether (4 × 50 ml) followed by drying and evaporation gave the *title compound* (0.35 g) as a foam. IR ($CHBr_3$) 3500, 3200 (br.), 1730, 1700 $cm^{-1}$. TLC (X) $R_f$ 025.

The following compounds were prepared by a similar procedure:

(b) *[1α(Z),2β,3α,5α] - (±) - 7 - [3 - Hydroxy - 5 - [4 - methylthio(phenylmethoxy) - 2 - (4 - morpholinyl)cyclopentyl] - 5 - heptenoic acid* from the product of Example 1b). Purification by chromatography (I).

| | | | |
|---|---|---|---|
| Analysis Found: | C, 63.5; | H, 8.1; | N, 3.0; |
| $C_{24}H_{35}NO_5S$ requires: | C, 64.1; | H, 7.9; | N, 3.1% |

(c) *[1α(Z),2β,3α,5α] - (±) - 7 - [3 - Hydroxy - 2 - (4 - morpholinyl) - 5 - [4 - (2 - phenylethyl)phenylmethoxy]cyclopentyl] - 5 - heptenoic acid*, IR (Neat) 3350, 1700 $cm^{-1}$, from the product of Example 1c).

(d) *[1α(Z),2β,3α,5α] - (±) - 7 - [3 - Hydroxy - 2 - (4 - morpholinyl) - 5 - (3 - thienylmethoxy)cyclopentyl] - 5 - heptenoic acid,* from the product of Example 11), IR ($CHBr_3$) 3500 (br), 1730, 1700 $cm^{-1}$, TLC ($SiO_2$) (Q) $R_f$ 0.13. Purification by chromatography (Q).

Example 3

(a) *[1α(Z),2β,3α,5α]-(±))-Methyl 7-[3-Hydroxy-5-[4-methylsulphonyl(phenylmethoxy)]-2-[4-morpholinyl)cyclopentyl]-5-heptenoate*

Peracetic acid (6.12 M, 0.49 ml) in acetic acid (10 ml) was added dropwise to a mixture of the product of Example 1b) (0.7 g) and $CH_3COONa$ (0.25 g) in acetic acid (15 ml) at 0°. After stirring for 2h saturated $Na_2SO_3$ solution was added and the suspension evaporated to dryness. The residue was neutralised with 8% $NaHCO_3$ solution, extracted with $CH_2Cl_2$ (3 × 75 ml), dried ($MgSO_4$), filtered and evaporated to afford an oil (0.83 g). Column chromatography (V) gave the *title compound* as an oil (0.4 g). TLC (V) $R_f$ 0.36.

(b) *[1α(Z),2β,3α,5α] - (±) - Methyl 7 - [3 - Hydroxy - 5 - [4 - methylsulphinyl(phenylmethoxy)] - 2 - (4 - morpholinyl)cyclopentyl] - 5 - heptenoate* was prepared from the product of Example 1b) (0.7 g) according to the method of Example 3a). Continued elution of the column using (V) gave the *title compound* as an oil (0.13 g). IR ($CHBr_3$) 3580/3440, 1723, 1040 $cm^{-1}$. TLC (V) Rf 0.2.

Example 4

(a) *[1α(Z),2β,3α,5α]-(±)-7-(3-Hydroxy-5-[[4'-methoxy(1,1'-biphenyl)-4-yl]methoxy]-2-(4-morpholinyl)-cyclopentyl]-5-heptenoic acid, hydrochloride*

To a solution of potassium t-butoxide (2.28 g) in THF (15 ml) under dry nitrogen was added (4-carboxybutyl)triphenylphosphonium bromide (4.5 g). After 15 min at 20° a solution of Intermediate 33 (1.6 g) in THF was added and stirring continued for a further 1h. Water (2 ml) was added and the THF removed *in vacuo*. The residue was taken up into water (100 ml), basified (pH 10) with NaOH solution and washed with ether (2 × 75 ml). The aqueous layer was adjusted to pH 6 with 2N hydrochloric acid, extracted with ether (5 × 75 ml), dried, evaporated and re-dissolved in EA (25 ml) and ether (40 ml). To the solution was added an excess of ethereal hydrogen chloride solution followed by cooling until crystallisation occurred. Filtration and purification from EA-methanol gave the *title compound* (1.2 g) m.p. 164—166°.

The following compounds were prepared by a similar procedure:

(b) *[1α(Z),2β,3α,5α] - (±) - 7 - [3 - Hydroxy - 2 - (4 - morpholinyl) - 5 - [4 - (phenylmethyl)phenylmethoxy]cyclopentyl] - 5 - heptenoic acid*, hydrochloride, m.p. 167—169° from Intermediate 33c).

(c) *[1α(Z),2β,3α,5α] - (±) - 7 - [3 - Hydroxy - 2 - (4 - morpholinyl) - 5 - [[(1,1';4',1'' - terphenyl) - 4 - yl]methoxy]cyclopentyl - 5 - heptenoic acid,* hydrochloride, m.p. 188—190° from Intermediate 33b).

(d) *[1α(Z),2β,3α,5α] - (±) - 7 - [3 - Hydroxy - 2 - (4 - morpholinyl) - 5 - [4 - phenylsulphonyl(phenylmethoxy)]cyclopentyl] - 5 - heptenoic acid,* from Intermediate 33e). Purification by chromatography (Q). (IR ($CHBr_3$), 1730 (sh), 1705 $cm^{-1}$, TLC (V) $R_f$ 0.27.

(e) *[1α(Z),2β,3α,5α] - (±) - 7 - [3 - Hydroxy - 2 - (4 - morpholinyl) - 5 - [4 - thien - 2 - yl)phenylmethoxy]cyclopentyl] - 5 - heptenoic acid,* from Intermediate 33d). Purification by chromatography (W) followed by (X). IR (Neat 3360, 1710 $cm^1$, TLC (X) $R_f$ 0.4.

(f) *[1α(Z),2β,3α,5α] - (±) - 7 - [3 - Hydroxy - 2 - (4 - morpholinyl) - 5 - [(5 - phenylthien - 2 - yl)methoxy]cyclopentyl] - 5 - heptenoic acid,* from Intermediate 34a), IR ($CHBr_3$), 3500, 1740, 1705 $cm^{-1}$, TLC ($SiO_2$) 92.5:7.5 $CH_2Cl_2$ — methanol $R_f$ 0.16.

(g) *[1α(Z),2β,3α,5α] - (±) - 7 - [5 - [(5 - Cyclohexylthien - 2 - yl)methoxy] - 3 - hydroxy - 2 - (4 - morpholinyl)cyclopentyl] - 5 - heptenoic acid,* from Intermediate 34b), IR (Neat 1720 $cm^{-1}$ (broad), TLC (Q) $R_f$0.33.

Example 5

(a) *[1α(Z),2β,5α]-(±)-7-[5-[[4'-Methyl(1,1'-biphenyl)-4-yl]methoxy]-2-(4-morpholinyl)-3-oxocyclopentyl]-5-heptenoic acid*

To a stirred solution of the product of Example 2 (0.393 g) in DMSO (3 ml) and $CH_2Cl_2$ (3 ml) containing triethylamine (0.95 ml) at −10° was added pyridine-$SO_3$ complex (0.4 g) in DMSO (3 ml). After 1.5 h at −10° to −5° a further portion of pyridine-$SO_3$ complex (0.3 g) was added and stirring continued for 0.5 h. The mixture was poured into water and the $CH_2Cl_2$ removed *in vacuo.* The aqueous solution was acidified to pH 6 with citric acid solution, extracted with EA (3 × 50 ml), washed with water, dried ($MgSO_4$) and concentrated, and the residue purified by chromatography (V) gave the *title compound* as an oil (0.154 g). IR ($CHBr_3$) 3490, 1740, 1703 $cm^{-1}$. TLC (K) $R_f$ 0.48.

The following compounds were prepared by a similar procedure:

(b) *[1α(Z),2β,5α] - (±) - 7 - [5 - [4 - Methylthio - (phenylmethoxy)] - 2 - (4 - morpholinyl) - 3 - oxocyclopentyl] - 5 - heptenoic acid* from the product of Example 2b). IR ($CHBr_3$) 3500, 1735, 1700 $cm^{-1}$. TLC (J) $R_f$ 0.29.

(c) *[1α(Z),2β,5α] - (±) - 7 - [2 - (4 - Morpholinyl - 3 - oxo - 5 - [4 - (2 - phenylethyl)phenylmethoxy]cyclopentyl] - 5 - heptenoic acid,* from the product of Example 2c). IR ($CHBr_3$) 3590, 3500, 1735, 1700 $cm^{-1}$. TLC (J) $R_f$ 0.31. Purification by chromatography (J).

(d) *[1α(Z),2β,5α] - (±) - Methyl 7 - [5 - [4 - Methylsulphonyl(phenylmethoxy)] - 2 - (4 - morpholinyl) - 3 - oxocyclopentyl] - 5 - heptenoate* from the product of Example 3b). IR ($CHBr_3$) 1735, 1300 $cm^{-1}$. Purification by chromatography (Q).

(e) *[1α(Z),2β,5α] - (±) - Methyl 7 - [5 - [4 - methylsulphinyl(phenylmethoxy)] - 2 - (4 - morpholinyl) - 3 - oxocyclopentyl] - 5 - heptenoate* from the product of Example 3b). IR ($CHBr_3$) 1730, 1040 $cm^{-1}$ TLC (I) $R_f$ 0.41. Purification by chromatography (I).

(f) *[1α(Z),2β,5α] - (±) - 7 - [2 - (4 - Morpholinyl) - 3 - oxo - 5 - [4 - (thien - 2 - yl)phenylmethoxy]cyclopentyl] - 5 - heptenoic acid,* from the product of Example 4e), m.p. 92° IR ($CHBr_3$) 3500, 1705 $cm^{-1}$. Purification by chromatography (T).

(g) *[1α(Z),2β,5α] - (±) - 7 - [5 - [(5 - Cyclohexylthien - 2 - yl)methoxy] - 2 - (4 - morpholinyl) - 3 - oxocyclopentyl] - 5 - heptenoic acid,* from the product of Example 4g). IR (Neat) 1740, 1710 $cm^{-1}$, TLC ($SiO_2$) (G) $R_f$ 0.27. Purification by chromatography (Z).

Example 6

*[1α(Z),2β,5α]-(±)-7-[2-(4-Morpholinyl)-3-oxo-5-(2-thienylmethoxy)cyclopentyl]-5-heptenoic acid*

A mixture of the product of Example 1j) (1.2 g) and KOH (0.3 g) in methanol (15 ml) and water (7.5 ml) was stirred for 5h. at room temperature. The methanol was removed *in vacuo* and the aqueous residue was diluted with water (20 ml) and acidified to pH 6.5 with $NaHSO_4$. Extraction with $CH_2Cl_2$ gave, after drying over $MgSO_4$ and concentrating, the crude acid as an oil (0.5 g). A solution of this acid was converted to the *title compound* according to the method of Example 5a). IR ($CHBr_3$) 3490, 1733, 1700 $cm^{-1}$. TLC (T) $R_f$ 0.2. Purification by chromatography (AB).

Example 7

(a) *[1α,2β,3α,5α]-(±)-3-Hydroxy-5-[[4'-methyl(1,1'-biphenyl)-4-yl]methoxy]-2-(4-morpholinyl)cyclopentane heptanoic acid*

A solution of the product of Example 2a) (0.78 g) in EA (35 ml) was hydrogenated at atmospheric

pressure over pre-reduced 10% palladium oxide on charcoal (0.4 g) at 20° for 4h. The mixture was filtered ('Hyflo') and the filtrate evaporated to give the *title compound* as an oil (0.75 g). IR ($CHBr_3$) 3580/3560, 3500, 1725, 1715 $cm^{-1}$. TLC (X) $R_f$ 0.4.

The following compounds were prepared by a similar procedure:

(b) *[1α,2β,3α,5α] - (±) - 3 - Hydroxy - 2 - (4 - morpholinyl) - 5 - [4 - phenylmethyl)phenylmethoxy]cyclopentane heptanoic acid* from the product of Example 4b). A sample was converted into the hydrochloride salt to give material of m.p. 125—128° (dec). IR (Nujol) 3300, 2800/2400, 1690 $cm^{-1}$.

(c) *[1α,2β,3α,5α] - (±) - 3 - Hydroxy - 5 - [(4' - methoxy(1,1' - biphenyl) - 4 - yl]methoxy] - 2 - (4 - morpholinyl)cyclopentane heptanoic acid*, from the free base of the product of Example 4a). IR ($CHBr_3$) 3500, 1725, 1703 $cm^{-1}$.

Example 8

*[1α(Z),2β,3α,5α]-(±)-Methyl 7-[3-Hydroxy-2-(4-morpholinyl)-5-[[4-(phenylmethyl)thien-2-yl]methoxy]cyclopentyl]-5-heptenoate.*

A solution of Intermediate 36a) (2 g) in 9:1 methanol-sulphuric acid (15 ml) was stirred for 2h at room temperature, whereupon $NaHCO_3$ solution (100 ml) was added. The mixture was extracted with EA (3 × 70 ml), the extracts washed with brine, dried ($MgSO_4$) and concentrated. The residue was chromatographed (I) to give the *title compound* as an oil (1.44 g). IR ($CHBr_3$) 3500—3400 (broad), 1728 $cm^{-1}$.

| | | | |
|---|---|---|---|
| Analysis Found: | C, 67.4; | H, 7.5; | N, 2.7. |
| $C_{29}H_{39}NO_5S$ requires: | C, 67.8; | H, 7.7; | N, 2.7%. |

Example 9

(a) *[1α(Z),2β,3α,5α]-(±)-7-[5-[4-N,N-Dimethylaminosulphonyl-(phenylmethoxy)]-3-hydroxy-2-(4-morpholinyl)cyclopentyl]-5-heptenoic acid*

A solution of Intermediate 38 (0.77 g) in methanol (30 ml) containing 2N NaOH (5 ml) was stirred at room temperature for 18h. The solution was treated with pH 6.5 buffer ($Na_2HPO_4/KH_2PO_4$) and extracted with $CH_2Cl_2$. The dried ($MgSO_4$) extracts were evaporated to give the *title compound* (0.612g) as a foam. IR ($CHBr_3$) 3580, 3500, 1730, 1703, 1340 $cm^{-1}$.

| | | | |
|---|---|---|---|
| Analysis Found: | C, 58.6; | H, 7.4; | N, 5.1. |
| $C_{25}H_{38}N_2O_7S$ requires: | C, 58.8; | H, 7.5; | N, 5.5% |

The following compounds were prepared using a similar procedure:

(b) *[1α(Z),2β,3α,5α] - (±) - 7 - [3 - Hydroxy - 5 - [[4' - methoxy(1,1' - biphenyl) - 4 - yl]methoxy] - 2 - (1 - piperidinyl)cyclopentyl] - 5 - heptenoic acid*, from the product of Example 1d), m.p. 94—101°.

| | | | |
|---|---|---|---|
| Analysis Found: | C, 73.0; | H, 8.1; | N, 2.6. |
| $C_{31}H_{41}NO_5$ requires: | C, 73.3; | H, 8.1; | N, 2.8% |

(c) *[1α(Z),2β,3α,5α] - (±) - 7 - [3 - Hydroxy - 5 - [4 - (phenylmethyl)phenylmethoxy] - 2 - (1 - piperidinyl)cyclopentyl] - 5 - heptenoic acid*, from the product of Example 1e), m.p. 103—110.5°. IR ($CHBr_3$) 3500, 1730—1700 (br) $cm^{-1}$.

(d) *[1α(Z),2β,3α,5α] - (±) - 7 - [3 - Hydroxy - 5 - [[4' - methyl(1,1' - biphenyl) - 4 - yl]methoxy] - 2 - (1 - piperidinyl)cyclopentyl] - 5 - heptenoic acid* from the product of Example 1f), m.p. 52—65°. TLC 3:1 Methanol-EA $R_f$ 0.26.

(e) *[1α(Z),2β,3α,5α] - (±) - 7 - [3 - Hydroxy - 5 - [[4' - methoxy(1,1' - biphenyl) - 4 - yl]methoxy] - 2 - (4 - thiomorpholinyl)cyclopentyl] - 5 - heptenoic acid, S-dioxide* from the product of Example 1g), IR ($CHBr_3$) 3500, 1735, 1705 $cm^{-1}$. TLC (I) $R_f$ 0.33.

(f) *[1α(Z),2β,3α,5α] - (±) - 7 - [3 - Hydroxy - 5 - [4 - (phenylmethyl)phenylmethoxy] - 2 - (4 - thiomorpholinyl)cyclopentyl] - 5 - heptenoic acid, S-dioxide* from the product of Example 1h), IR ($CHBr_3$) 3580, 3500, 1733, 1710 $cm^{-1}$. TLC (Q) $R_f$ 0.35.

(g) *[1α(Z),2β,3α,5α] - (±) - 7 - [3 - Hydroxy - 5 - [[4' - methyl(1,1' - biphenyl) - 4 - yl]methoxy - 2 - (4 - thiomorpholinyl)cyclopentyl] - 5 - heptenoic acid, S-dioxide* from the product of Example 1i), IR ($CHBr_3$) 3600 (br.), 3500, 1740, 1710 $cm^{-1}$. TLC (I) $R_f$ 0.36.

(h) *[1α(Z),2β,3α,5α] - (±) - 7 - [3 - Hydroxy - 2 - (4 - morpholinyl) - 5 - [(4 - phenylthien - 2 - yl)methoxy]cyclopentyl] - 5 - heptenoic acid* from the product of Example 1o), m.p. 119.5—121.5°.

(i) *[1α(Z),2β,3α,5α] - (±) - 7 - [3 - Hydroxy - 2 - (4 - morpholinyl) - 5 - [[4 - (phenylmethyl)thien - 2 - yl]methoxy]cyclopentyl] - 5 - heptenoic acid,* from the product of Example 8. Purification by chromatography (L), IR (Neat) 3370, 1705 $cm^{-1}$, TLC (I) $R_f$ 0.29.

(j) *[1α(Z),2β,3α,5α] - (±) - 7 - [3 - Hydroxy - 5 - [(4 - methylthien - 2 - yl)methoxy] - 2 - (4 - morpholinyl)cyclopentyl] - 5 - heptenoic acid,* from the product of Example 1m), IR ($CHBr_3$) 3600—3500 broad, 1730, 1705 $cm^{-1}$, TLC (I) $R_f$ 0.23.

Example 10

(a) *[1α(Z),2β,3α,5α]-(±)-7-[5-[(4-Bromothien-2-yl)methoxy]-3-hydroxy-2-(4-morpholinyl)cyclopentyl]-5-heptenoic acid.*

A stirred solution of Intermediate 36b) (1.29 g) in methanol (100 ml) was treated with concentrated sulphuric acid (3.5 ml) and the mixture stirred at room temperature for 1h. After quenching by carefully pouring into 8% $NaHCO_3$ solution (250 ml) the methanol was removed and the residue extracted with EA (3 × 50 ml). The combined extracts were evaporated and the residue was dissolved in a mixture of 2N NaOH (5 ml) and methanol (10 ml) with stirring at room temperature. After 18h the mixture was evaporated *in vacuo* and the residue acidified to pH 6.5 with 1M aqueous $KH_2PO_4$. Extraction with EA (3 × 50 ml), drying ($MgSO_4$) and evaporation gave the *title compound* as a glass (0.98 g). IR ($CHBr_3$) 3580, 3560, 3500, 1725, 1700 $cm^{-1}$. TLC (X) $R_f$ 0.22.

(b) *[1α(Z),2β,3α,5α] - (±) - 7 - [3 - Hydroxy - 5 - [[4 - (4 - methoxyphenyl)thien - 2 - yl]methoxy] - 2 - (4 - morpholinyl)cyclopentyl] - 5 - heptenoic acid,* was similarly prepared from Intermediate 36c), m.p. 95—100°.

| | | | |
|---|---|---|---|
| Analysis Found: | C, 65.6; | H, 7.4; | N, 2.7. |
| $C_{28}H_{37}NO_6S$ requires: | C, 65.2; | H, 7.2; | N, 2.7%. |

Example 11

(a) *[1α(Z),2β,5α]-(±)-7-[5-[[4'-Methoxy(1,1'-biphenyl)-4-yl]methoxy]-2-(4-morpholinyl)-3-oxocyclopentyl]-5-heptenoic acid*

To a solution of the product of Example 4a) (1.3 g) and triethylsilyl chloride (0.56 ml) in $CH_2Cl_2$ (8 ml) at 0° was added triethylamine (0.475 ml) and the mixture stirred at 0° for 15 min. Simultaneously, a solution of oxalyl chloride (0.68 ml) in $CH_2Cl_2$ (8 ml) was cooled to −70° under nitrogen and DMSO (1.35 ml) added dropwise. After stirring for 10 min. the solution of triethylsilyl ester described above was added and stirring continued at −70° for 0.75h. Triethylamine (4 ml) was added and the cooling bath removed. When room temperature was attained the mixture was poured into $NH_4Cl$ solution (100 ml) and extracted with ether (3 × 70 ml). The combined extracts were evaporated to leave an oily residue which was stirred with $KH_2PO_4$ (1 g) in acetone (5 ml), water (20 ml) and ether (5 ml) at 20° for 1.5 h. After dilution with water (100 ml) the mixture was extracted with ether (2 × 100 ml), dried and evaporated to give an oil (1.71 g). A portion (1.25 g) was chromatographed (J) to give an oil which crystallised from ether-isopentane at −20° to give the *title compound* (0.175 g), m.p. 79—82°,

| | | | |
|---|---|---|---|
| Analysis Found: | C, 70.7; | H, 7.2; | N, 2.8. |
| $C_{30}H_{37}NO_6$ requires: | C, 71.0; | H, 7.4; | N, 2.8% |

The following compounds were prepared by a similar procedure:

(b) *[1α(Z),2β,5α] - (±) - 7 - [2 - (4 - Morpholinyl) - 3 - oxo - 5 - [[4 - (phenylmethyl)phenyl]methoxy]cyclopentyl] - 5 - heptenoic acid,* m.p. 68—71° (from ether-isopentane) from the free base of the product of Example 4b).

(c) *[1α(Z),2β,5α] - (±) - 7 - [2 - (4 - Morpholinyl) - 3 - oxo - 5 - [[(1,1';4.1'' - terphenyl) - 4 - yl]methoxy]cyclopentyl] - 5 - heptenoic acid,* m.p. 126—128° (dec.) (from EA—PE (b.p. 60—80°)) from the product of Example 4c). Purification by chromatography (AB).

(d) *[1α,2β,5α] - (±) - 2 - (4 - Morpholinyl) - 3 - oxo - 5 - [4 - (phenylmethyl)phenylmethoxy]cyclopentaneheptanoic acid* from the product of Example 7b), mp 83—85°. Purification by chromatography (J) IR (Nujol) 1740, 1718 $cm^{-1}$

(e) *[1α(Z),2β,5α] - (±) - 7 - [5 - [[4' - Methoxy(1,1' - biphenyl) - 4 - yl]methoxy] - 3 - oxo - 2 - (1 - piperidinyl)cyclopentyl] - 5 - heptenoic acid,* from the product of Example 9b) except that trimethylsilyl chloride and toluene were used instead of triethylsilyl chloride and $CH_2Cl_2$.

Purification by chromatography (J) IR (Nujol) 1740, 1710 $cm^{-1}$

| | | | |
|---|---|---|---|
| Analysis Found: | C, 73.7; | H, 8.0; | N, 2.8. |
| $C_{31}H_{39}NO_5$ requires: | C, 73.6; | H, 7.8; | N, 2.8% |

(f) *[1α(Z),2β,5α] - (±) - 7 - [3 - Oxo - 5 - [4 - (phenylmethyl)phenylmethoxy] - 2 - (1 - piperidinyl)cyclopentyl] - 5 - heptenoic acid,* from the product of Example 9c. Purification by chromatography (AC). IR (Neat) 2800—2500, 1735, 1710 $cm^{-1}$. TLC (J) $R_f$ 0.32.

(g) *[1α(Z),2β,5α] - (±) - 7 - [5 - [[4' - Methyl(1,1' - biphenyl) - 4 - yl]methoxy] - 3 - oxo - 2 - (1 - piperidinyl)cyclopentyl] - 5 - heptenoic acid,* from the product of Example 9d. Purification by chromatography (J). IR ($CHBr_3$) 3500, 1735, 1700 $cm^{-1}$. TLC (J) $R_f$ 0.27.

(h) *[1α(Z),2β,5α] - (±) - 7 - [5 - [[4' - Methoxy(1,1' - biphenyl) - 4 - yl]methoxy] - 3 - oxo - 2 - (4 - thiomorpholinyl)cyclopentyl] - 5 - heptenoic acid, S-dioxide,* from the product of Example 9e). Purification by chromatography (AD) IR ($CHBr_3$) 3450, 1740, 1700 $cm^{-1}$. TLC (I) $R_f$ 0.38.

(i) *[1α(Z),2β,5α] - (±) - 7 - [3 - Oxo - 5 - [4 - (phenylmethyl)phenylmethoxy] - 2 - (4 - thiomorpholinyl)cyclopentyl] - 5 - heptenoic acid, S-dioxide,* from the product of Example 9f) m.p. 100—101.5°. IR ($CHBr_3$) 3490, 1745, 1710 $cm^{-1}$. Purification by chromatography (J).

(j) *[1α(Z),2β,5α] - (±) - 7 - [5 - [[4' - Methyl(1.1 - Methyl(1,1' - biphenyl) - 4 - yl]methoxy] - 3 - oxo - 2 - (4 - thiomorpholinyl)cyclopentyl] - 5 - heptenoic acid, S-dioxide,* from the product of Example 9g). Purification by chromatography (J). IR ($CHBr_3$) 3480, 1740, 1700 $cm^{-1}$ TLC (I) $R_f$ 0.5.

The following compounds were prepared by a similar procedure except that trimethylsilyl chloride and toluene were used instead of triethylsilyl chloride and $CH_2Cl_2$ respectively:

(k) *[1α(Z),2β,5α] - (±) - 7 - [2 - (4 - Morpholinyl) - 3 - oxo - 5 - [(4 - phenylthien - 2 - yl)methoxy]cyclopentyl] - 5 - heptenoic acid,* from the product of Example 9h), m.p. 114—116°. Purification by chromatography (J).

(l) *[1α(Z),2β,5α] - (±) - 7 - [2 - (4 - Morpholinyl) - 3 - oxo - 5 - (3 - thienylmethoxy)cyclopentyl] - 5 - heptenoic acid,* from the product of Example 2d). Purification by chromatography (H). IR ($CHBr_3$) 3500, 3200—2300 (broad), 1735, 1700 $cm^{-1}$. TLC (Q) $R_f$ 0.2.

(m) *[1α(Z),2β,5α] - (±) - 7 - [2 - (4 - Morpholinyl) - 3 - oxo - 5 - [[4 - (phenylmethyl)thien - 2 - yl]methoxy]cyclopentyl] - 5 - heptenoic acid,* from the product of Example 9i). Purification by chromatography (AE). IR ($CHBr_3$) 1738, 1700 $cm^{-1}$ TLC. (J) $R_f$ 0.3.

(n) *[1α(Z),2β,5α] - (±) - 7 - [5 - [(4 - Bromothien - 2 - yl)methoxy] - 2 - (4 - morpholinyl) - 3 - oxocyclopentyl] - 5 - heptenoic acid,* from the product of Example 10a). Purification by chromatography (AF). IR ($CHBr_3$) 1738, 1700 $cm^{-1}$ TLC (J) $R_f$ 0.19.

(o) *[1α(Z),2β,5α] - (±) - 7 - [5 - [[4 - (4 - Methoxyphenyl)thien - 2 - yl]methoxy] - 2 - (4 - morpholinyl) - 3 - oxocyclopentyl] - 5 - heptenoic acid,* from the product of Example 10b). Purification by chromatography (AF). m.p. 107.5—109°.

| | | | |
|---|---|---|---|
| Analysis Found: | C, 65.6; | H, 6.9; | N, 2.6. |
| $C_{28}H_{35}NO_6$ requires: | C, 65.5; | H, 6.9; | N, 2.7%. |

(p) *[1α(Z),2β,5α] - (±) - 7 - [5 - [(4 - Methylthien - 2 - yl)methoxy] - 2 - (4 - morpholinyl) - 3 - oxocyclopentyl] - 5 - heptenoic acid,* from the product of Example 9j). Purification by chromatography (J). IR ($CHBr_3$) 3500, 1735, 1700 $cm^{-1}$. TLC (L) $R_f$ 0.4.

Example 12

*[1α(E),2β,3α,5α]-(±)-7-[3-Hydroxy-5-[[4'-methyl(1,1'-biphenyl)-4-yl]methoxy]-2-(4-morpholinyl)cyclopentyl]-5-heptenoic acid*

A solution of the product of Example 2a) (0.28 g) and p-toluene sulphinic acid (0.133 g) in dry 1,4-dioxan (20 ml) was heated under reflux under nitrogen for 3h. After cooling, EA (25 ml) was added, the solution washed with aqueous pH 6 phosphate buffer (30 ml), dried and concentrated. Purification by chromatography (X) EA-methanol gave the *title compound* as an oil (0.228 g). IR ($CHBr_3$) 3580, 3500, 1720, 1705 $cm^{-1}$.

Example 13

*[1α(Z),2β,3α,5α]-(±)-7-[5-[[4'-Chloro(1,1'-biphenyl)-4-yl]methoxy]-3-hydroxy-2-(4-morpholinyl)cyclopentyl]-5-heptenoic acid*

A solution of Intermediate 40 (1.7 g) in 10% concentrated sulphuric acid in methanol (30 ml) was stirred for 16h. The solution was neutralised with 8% $NaHCO_3$solution and extracted into $CH_2Cl_2$. The extracts were evaporated and the residue then dissolved in a solution of KOH (0.4 g) in methanol (20 ml) and water (10 ml). After 24h at room temperature, the solution was treated with $NaHSO_4$ solution until pH 6.5, whereupon the mixture was extracted with $CH_2Cl_2$. The dried ($MgSO_4$) extracts were concentrated and the residue purified by chromatography (AG) followed by (AH) to give the *title compound* as a foam (0.45 g). IR ($CHBr_3$) 3600—3440, 1730, 1700 $cm^{-1}$. TLC 9:1 Chloroform-methanol $R_f$ 0.7.

Example 14

*[1α(Z),2β,5α]-(±)-7-[2-(4-Morpholinyl)-3-oxo-5-[(5-phenylfuran-2-yl)methoxy]cyclopentyl]-5-heptenoic acid*

Trimethylsilyl chloride (0.073 ml) was added to a solution of the product of Example 1n) (0.25 g) and triethylamine (0.082 ml) in dry toluene (5 ml) at 0°. After stirring for 5 min. the mixture was then added to a mixture of N-chloro-succinimide (0.178 g) and dimethylsulphide (0.107) in dry toluene (10 ml) and stirring continued for 45 min. Triethylamine (0.28 ml) was added followed after 5 min. by water (10 ml) and $KH_2PO_4$ solution until pH 6. The mixture was extracted with ether (3 × 20 ml) and the combined extracts washed with brine (50 ml) and dried ($MgSO_4$). Filtration and evaporation gave an oil which was purified by flash chromatography (AI). The *title compound* was obtained as a solid (0.084 g), which crystallised from ether, m.p. 91.5—92.5°.

Example 15

*[1α(Z),2β,3α,5α]-(±)-Methyl 7-[5-[4-Acetylamino(phenylmethoxy)]-3-hydroxy-2-(4-morpholinyl)cyclopentyl]-5-heptenoate*

Anhydrous $K_2CO_3$ (0.21 g) was added to a stirred solution of Intermediate 35 (0.61 g) in dry methanol (20 ml). After 2.5h, the suspension was poured into saturated $NH_4Cl$ solution (50 ml), extracted with $CH_2Cl_2$ (3 × 30 ml), dried ($MgSO_4$), filtered and evaporated, and the residue purified by column chromatography (AJ) to give the *title compound* as an oil (0.5 g). IR ($CHBr_3$) 3500 (br), 3420, 1725, 1685, 1510 $cm^{-1}$. TLC (AJ) Rf 0.38.

Example 16

*[1α(Z),2β,5α]-(±)-Methyl 7-[5-[4-Acetylamino(phenylmethoxy)]-2-(4-morpholinyl)-3-oxocyclopentyl]-5-heptenoate*

Pyridinium trifluoroacetate (0.21 g) was added to a stirred mixture of the product of Example 15 (0.35 g) and dicyclohexylcarbodiimide (0.61 g) in dry DMSO (6 ml). After 30 min. the suspension was poured into water (50 ml), extracted with ether (3 × 40 ml), dried ($MgSO_4$), filtered and evaporated to afford an oil, which was purified by column chromatography (AK) to give the *title compound* as a solid (0.3 g) m.p. 88—88.5°.

Example 17

(a) *[1α(Z),2β,5α]-(±)-Methyl 7-[5-[4-Methylthio(phenylmethoxy)]-2-(4-morpholinyl)-3-oxocyclopentyl]-5-heptenoate*

A solution of the product of Example 5b) (0.5 g) in ether (10 ml) was treated with an excess of ethereal $CH_2N_2$ at room temperature. Excess $CH_2N_2$ was destroyed by the addition of acetic acid. The solution was diluted with water and washed with 8% $NaHCO_3$ solution followed by water. Evaporation of the dried solvent afforded an oil which was purified by chromatography (G) to give the *title compound* (0.3 g) IR ($CHBr_3$) 1735 $cm^{-1}$.

The following compounds were prepared by a similar procedure:

(b) *[1α(Z),2β,5α] - (±) - Methyl 7 - [5 - [[4' - Methyl(1,1' - biphenyl) - 4 - yl]methoxy] - 2 - (4 - morpholinyl) - 3 - oxocyclopentyl] - 5 - heptenoate*, m.p. 82—86° (from ether-PE (b.p. 60—80°)) from the product of Example 5a),

(c) *[1α(Z),2β,5α] - (±) - Methyl 7 - [5 - [[4' - Methoxy(1,1' - biphenyl) - 4 - yl]methoxy] - 2 - (4 - morpholinyl) - 3 - oxocyclopentyl] - 5 - heptenoate*, m.p. 79—84° (from EA—PE (b.p. 60—80°)) from the product of Example 11a).

(d) *[1α(Z),2β,5α] - (±) - Methyl 7 - [2 - (4 - Morpholinyl - 3 - oxo - 5 - [[[4 - (phenylmethyl)phenyl] - 4 - yl]methoxy]cyclopentyl] - 5 - heptenoate,* m.p. 30—33° (from ether-isopentane at −20° from the product of Example 11b).

Example 18

(a) *(1α,2β,5α)-(±)-5-[[4′-Methyl(1,1′-biphenyl)-4-yl]methoxy]-2-(4-morpholinyl)-3-oxocyclopentaneheptanoic acid*

A stirred solution of the product of Example 7a) (0.695 g) in acetone (20 ml) was cooled to −10° and Jones reagent (2.67 M, 0.75 ml) added dropwise. After 1.25h at −10° to −5° a further aliquot of Jones reagent (0.15 ml) was added and stirring continued for a further 0.5h, whereupon the mixture was poured into pH 6.5 phosphate buffer (100 ml) and extracted with $CH_2Cl_2$ (3 × 30 ml), dried ($MgSO_4$), filtered and evaporated, and the residue purified by chromatography (J) followed by (AL) to give the *title compound* which crystallised from ether-EA—PE (b.p. 60—80°) as needles (0.345 g), m.p. 88—89°.

| | | | |
|---|---|---|---|
| Analysis found: | C, 72.5; | H, 7.7; | N, 2.6. |
| $C_{30}H_{39}NO_5$ requires: | C, 73.0; | H, 8.0; | N, 2.8%. |

The following compounds were prepared using a similar procedure:

(b) *[1α(Z),2β,5α] - (±) - 7 - [2 - (4 - Morpholinyl) - 3 - oxo - 5 - [4 - phenylsulphonyl(phenylmethoxy)]cyclopentyl] - 5 - heptenoic acid,* from the product of Example 4d). Purification by chromatography (J) IR ($CHBr_3$) 3490, 1730, 1730, 1700, 1070 $cm^{-1}$. TLC (J) $R_f$ 0.16.

(c) *[1α(E),2β,5α] - (±) - 7 - [5 - [[4′ - Methyl(1,1′ - biphenyl) - 4 - yl]methoxy] - 2 - (4 - morpholinyl) - 3 - oxocyclopentyl] - 5 - heptenoic acid,* from the product of Example 12. Purification by chromatography (J). m.p. 82—93°.

| | | | |
|---|---|---|---|
| Analysis found: | C, 73.0; | H, 7.6; | N, 2.6. |
| $C_{30}H_{37}NO_5$ requires: | C, 73.3; | H, 7.6; | N, 2.9%. |

(d) *(1α,2β,5α) - (±) - 5 - [[4′ - Methoxy(1,1′ - biphenyl) - 4 - yl] - 2 - (4 - morpholinyl) - 3 - oxocyclopentane heptanoic acid,* from the product of Example 7c) m.p. 87—90°.

| | | | |
|---|---|---|---|
| Analysis found: | C, 70.7; | H, 8.1; | N, 3.0. |
| $C_{30}H_{39}NO_6$ requires: | C, 70.7; | H, 7.7; | N, 2.8%. |

(e) *[1α(Z),2β,5α] - (±) - 7 - [5 - [4 - N,N - Dimethylaminosulphonyl(phenylmethoxy)] - 2 - (4 - morpholinyl) - 3 - oxocyclopentyl] - 5 - heptenoic acid,* from the product of Example 9a). Purification by chromatography (AL). IR ($CHBr_3$) 3480, 1738, 1700, 1340 $cm^{-1}$.

| | | | |
|---|---|---|---|
| Analysis found: | C, 58.9; | H, 7.1; | N, 5.3. |
| $C_{25}H_{36}N_2O_7S$ requires: | C, 59.0; | H, 7.1; | N, 5.5%. |

(f) *[1α(Z),2β,5α] - (±) - 7 - [5 - [[4′ - Chloro(1,1′ - biphenyl) - 4 - yl]methoxy] - 2 - (4 - morpholinyl) - 3 - oxocyclopentyl] - 5 - heptenoic acid,* from the product of Example 13. Purification by chromatography (AG) followed by (T). IR ($CHBr_3$) 3500, 1740, 1700 $cm^{-1}$. TLC 95:5 chloroform-methanol $R_f$ 0.4.

(g) *[1α(Z),2β,5α] - (±) - 7 - [2 - (4 - Morpholinyl) - 3 - oxo - 5 - [(5 - phenylthien - 2 - yl)methoxy]cyclopentyl] - 5 - heptenoic acid,* from the product of Example 4f). IR ($CHBr_3$) 3500, 1740, 1705 $cm^{-1}$. TLC 92.5:7.5 $CH_2Cl_2$-methanol $R_f$ 0.35. Purification by chromatography (F) followed by (AM).

Example 19

*[1α(Z),2β,5α]-(±)-7-[2-(4-Morpholinyl)-3-oxo-5-[(2-phenylthien-4-yl)methoxy]cyclopentyl]-5-heptenoic acid*

The product of Example 1k) (0.9 g) was stirred with 2N NaOH (5 ml) in methanol (10 ml) for 3h. The methanol was removed *in vacuo* and $KH_2PO_4$ solution added until pH 6.5. The mixture was then extracted with EA (3 × 15 ml), washed with brine, dried ($MgSO_4$) and concentrated. The product (0.94 g) was

oxidised according to the method of Example 11a), except that trimethylsilyl chloride and toluene was used instead of triethylsilyl chloride and $CH_2Cl_2$ respectively, to give the *title compound* (0.31 g), m.p. 111—112°.

Pharmaceutical Examples

Tablets

| Direct Compression | Mg/tablet |
|---|---|
| Active ingredient | 100.00 |
| Microcrystalline Cellulose B.P.C | 298.00 |
| Magnesium Stearate | 2.00 |
| Compression Weight | 400.00 |

The active ingredient is sieved through a 250 $m^{-6}$ sieve, blended with the excipients and compressed using 10.0 mm punches. Tablets of other strengths may be prepared by altering the compression weight and using punches to suit.

Injection for Intravenous

| Administration | %w/v |
|---|---|
| Active ingredient | 0.50 |
| Water for injections B.P. to | 100.00 |

Sodium chloride may be added to adjust the tonicity of the solution and the pH may be adjusted to that of maximum stability and/or to facilitate solution of the active ingredient using either dilute acid or alkali.

The solution is prepared, clarified and filled into appropriate sized ampoules sealed by fusion of the glass. The injection is sterilised by heating in an autoclave using one of the acceptable cycles. Alternatively the solution may be sterilised by filtration and filled into sterile ampoules under aseptic conditions. The solution may be packed under an inert atmosphere of nitrogen.

| Inhalation Cartridges | /cartridge |
|---|---|
| Active ingredient (micronised) | 3 mg |
| Lactose B.P. to | 25 mg |

The active ingredient is micronised so that the majority of the particles are between 1 $m^{-6}$ and 5 $m^{-6}$ in longest dimension and none are greater than 10 $m^{-6}$. The active ingredient is then blended with the lactose and the mix is filled into No. 3 hard gelatin capsules using a suitable filling machine.

Potential Bronchodilatation

The following compounds were tested at concentrations between $2 \times 10^{-7}$ to $2 \times 10^{-5}$ mole/litre according to the method of K.M. Lulich *et al* in British Journal of Pharmacology *58*, 71—79, (1976), except guinea-pig lung was used instead of cat lung. $pA_2$ values were determined using the standard procedures of Arunlakshana and Schild in Br. J. Pharmac. Chemother, *14*, 48—58 (1959):

| Example No. | $pA_2$ | Example No. | $pA_2$ |
|---|---|---|---|
| 10b) | 9.0 | 11i) | 8.5 |
| 11e) | 8.8 | 18a) | 8.6 |
| 11g) | 8.5 | 18d) | 8.9 |

Inhibition of Platelet Aggregation

The following compounds were tested at a concentration of 2 µg/ml for their ability to inhibit the aggregation of human whole blood according to the method of G V Born in Nature *194*, 927—929 (1962) except collagen was used instead of ADP as the pro-aggregatory agent. $pA_2$ values were determined using the standard procedures of Arunlakshana and Schild in Br J Pharmac Chemother, *14*, 48—58 (1959):

| Example No. | $pA_2$ | Example No. | $pA_2$ |
|---|---|---|---|
| 5a) | 7.8 | 11a) | 7.8 |
| 5f) | 7.35 | 11b) | 7.4 |

Toxicity

The compounds are in general non-toxic at therapeutically useful doses. Thus for example the compounds of Examples 11a), 11c), and 11f) when given orally to dogs at a dose of 1 mg/kg produced no adverse effects.

**Claims for the Contracting States: BE CH DE FR GB IT LI NL SE**

1. Prostanoids of the general formula (1)

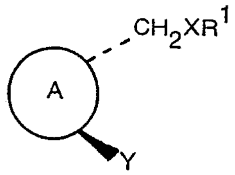

(1)

in which

A represents

(a) 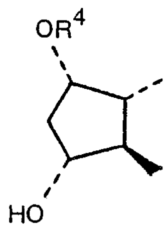 or (b) 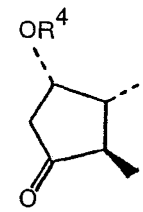

X is cis or trans $—CH=CH—$ or $—(CH_2)_2—$;

$R^1$ is straight or branched $C_{1-7}$ alkyl bearing as a terminal substituent $—COOR^{10}$ where $R^{10}$ is a hydrogen atom, $C_{1-6}$ alkyl or $C_{7-10}$ phenalkyl;

Y is a saturated heterocyclic amino group which has 5—8 ring members and (a) optionally contains in the ring —O—, —S—, $—SO_2$, $—NR^{14}—$ (where $R^{14}$ is a hydrogen atom, $C_{1-7}$ alkyl or phenalkyl having a $C_{1-4}$ alkyl portion), $>C(OH)R^6$ (where $R^6$ is a hydrogen atom, $C_{1-7}$ alkyl, phenyl, or phenalkyl having a $C_{1-4}$ alkyl portion); and/or (b) is optionally substituted by one or more $C_{1-4}$ alkyl groups;

$R^4$ is (i) phenalkyl (having a $C_{1-3}$ alkyl portion and the phenyl portion being substituted by $C_{1-3}$ alkylthio, $C_{1-3}$ alkylsulphinyl, $C_{1-3}$ alkylsulphonyl, $C_{1-3}$ alkanoylamino, benzoylamino, phenylalkyl having a $C_{1-3}$ alkyl portion, aminosulphonyl (the amino group being optionally substituted by one or two $C_{1-3}$ alkyl groups), $C_{1-3}$ alkanoylaminosulphonyl (the amino group being optionally substituted by $C_{1-3}$ alkyl), phenylsulphonyl (the phenyl portion being optionally substituted by $C_{1-3}$ alkyl), nitro, tetrazol-5-yl, phenyl substituted by $R^5$ (where $R^5$ is $C_{1-4}$ alkyl, $C_{1-4}$ alkoxy, halogen or phenyl), or thienyl);

or (ii) the group:

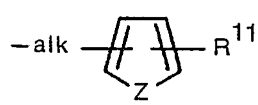

where alk is $C_{1-3}$ alkylene;

Z is O, or S;

$R^{11}$ is a hydrogen atom; $C_{1-6}$ alkyl; $C_{1-6}$ alkoxy; phenyl or phenylalkoxy or phenylalkyl having a $C_{1-3}$ alkyl portion (the phenyl portion in each case being optionally substituted by $C_{1-3}$ alkyl, $C_{1-3}$ alkoxy or halogen); phenyloxy; $C_{5-7}$ cycloalkyl; halogen or nitro; and the physiologically acceptable salts thereof.

2. Compounds as claimed in claim 1 in which A is the group (b).

3. Compounds as claimed in claim 1 or claim 2 in which Y is morpholino, dioxothiamorpholino or piperidino.

4. Compounds as claimed in any one of the preceding claims in which X is cis —CH=CH—.

5. Compounds as claimed in any one of the preceding claims in which $R^1$ is $-(CH_2)_3COOR^{10}$ where $R^{10}$ is a hydrogen atom or methyl.

6. Compounds as claimed in any one of the preceding claims in which $R^4$ is benzyl substituted by phenyl-($C_{1-3}$) alkyl or phenyl substituted by $C_{1-3}$ alkoxy or $C_{1-3}$ alkyl, or is thienylmethyl substituted by phenyl or ($C_{1-3}$ alkoxy) phenyl.

7. Compounds as claimed in claim 1 in which:

A is the group (b),

X is cis —CH=CH;

$R^1$ is $-(CH_2)_3COOH$,

Y is morpholino, piperidino or dioxothiamorpholino, and

$R^4$ is benzyl substituted by phenethyl, benzyl, methoxyphenyl or methylphenyl, or is methoxyphenylthienylmethyl.

8. Compounds as claimed in any one of the preceding claims, other than those in which $R^4$ is phenalkyl substituted by thienyl, substituted aminosulphonyl or substituted alkanoylaminosulphonyl.

9. A pharmaceutical composition comprising a compound as claimed in any one of the preceding claims together with one or more pharmaceutical carriers.

10. A process for the preparation of a compound as claimed in claim 1 which comprises:

(a) in the preparation of compounds of formula (2)

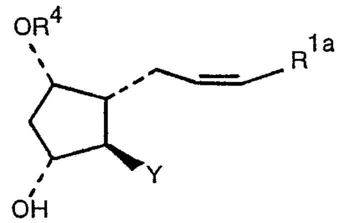

(2)

(where Y and $R^4$ are as defined in claim 1 and $R^{1a}$ is as defined for $R^1$ where $R^{10}$ is a hydrogen atom), reacting a compound of formula (3) or (3a)

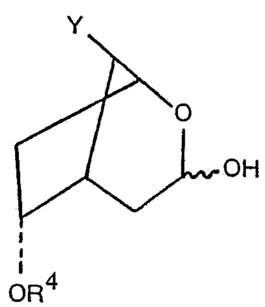

(3)

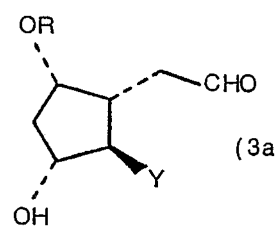

(3a)

with a phosphorane $R_3^{12}P = CHR^{1a}$ or a salt thereof (where $R^{12}$ is $C_{1-6}$ alkyl or phenyl);

(b) in the preparation of a compound in which A is (b), oxidising a compound as defined in claim 1 in which A is (a)

(c) esterifying a compound as defined in claim 1 in which $R^{10}$ is hydrogen to prepare a compound in which $R^{10}$ is alkyl or phenalkyl;

(d) saponifying a corresponding ester to prepare a compound as defined in claim 1 in which $R^{10}$ is hydrogen;

(e) isomerising a compound as defined in claim 1 in which A is the group (a) and X is cis —CH=CH— to prepare the corresponding trans compound;

(f) catalytically hydrogenating a compound as defined in claim 1 in which X is —CH=CH— to produce a compound in which X is $-(CH_2)_2-$;

(g) etherifying a compound as defined in claim 1 in which A is the group (a) and $-OR^4$ represents hydroxy, any other hydroxy group present being protected during the reaction;

(h) in the preparation of a compound as defined in claim 1 in which A is (a), removing the protecting group from a corresponding compound in which the ring hydroxy group is protected;

(i) in the preparation of a compound as defined in claim 1 in which $R^4$ is phenalkyl substituted by alkanoylamino, acylating the corresponding amine;

(j) in the preparation of a compound as defined in claim 1 in which A is the group (a) and $R^4$ is phenalkyl substituted by alkylsulphinyl or alkylsulphonyl, oxidising the corresponding alkylthio compound;

(k) in the preparation of a compound as defined in claim 1 in which A is the group (a) and Y is a

saturated heterocyclic amino group, treating the corresponding compound in which Y is $-NH_2$ with a difunctional reagent to form said heterocyclic amino group; or

(l) treating a compound as defined in claim 1 with an acid or (where $R^{10}$ is hydrogen) a base to form a salt.

**Claims for the Contracting State: AT**

1. A process for the preparation of prostanoids of the general formula (1)

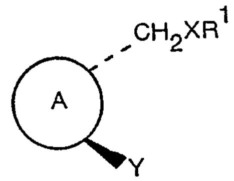

(1)

in which

A represents

(a) 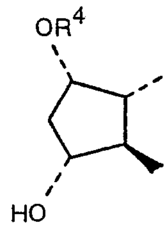 or (b) 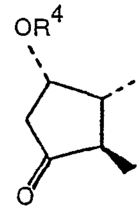

X is cis or trans $-CH{=}CH-$ or $-(CH_2)_2-$;

$R^1$ is straight or branched $C_{1-7}$ alkyl bearing as a terminal substituent $-COOR^{10}$ where $R^{10}$ is a hydrogen atom, $C_{1-6}$ alkyl or $C_{7-10}$ phenalkyl;

Y is a saturated heterocyclic amino group which has 5—8 ring members and (a) optionally contains in the ring $-O-$, $-S-$, $-SO_2$, $-NR^{14}-$ (where $R^{14}$ is a hydrogen atom, $C_{1-7}$ alkyl or phenalkyl having a $C_{1-4}$ alkyl portion), $>C(OH)R^6$ (where $R^6$ is a hydrogen atom, $C_{1-7}$ alkyl, phenyl, or phenalkyl having a $C_{1-4}$ alkyl portion); and/or (b) is optionally substituted by one or more $C_{1-4}$ alkyl groups;

$R^4$ is (i) phenalkyl (having a $C_{1-3}$ alkyl portion and the phenyl portion being substituted by $C_{1-3}$ alkylthio, $C_{1-3}$ alkylsulphinyl, $C_{1-3}$ alkylsulphonyl, $C_{1-3}$ alkanoylamino, benzoylamino, phenylalkyl having a $C_{1-3}$ alkyl portion, aminosulphonyl (the amino group being optionally substituted by one or two $C_{1-3}$ alkyl groups), $C_{1-3}$ alkanoylaminosulphonyl (the amino group being optionally substituted by $C_{1-3}$ alkyl), phenylsulphonyl (the phenyl portion being optionally substituted by $C_{1-3}$ alkyl), nitro, tetrazol-5-yl, phenyl substituted by $R^5$ (where $R^5$ is $C_{1-4}$ alkyl, $C_{1-4}$ alkoxy, halogen or phenyl), or thienyl);

or (ii) the group:

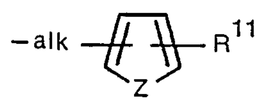

where alk is $C_{1-3}$ alkylene;

Z is O, or S;

$R^{11}$ is a hydrogen atom; $C_{1-6}$ alkyl; $C_{1-6}$ alkoxy; phenyl or phenylalkoxy or phenylalkyl having a $C_{1-3}$ alkyl portion (the phenyl portion in each case being optionally substituted by $C_{1-3}$ alkyl, $C_{1-3}$ alkoxy or halogen); phenyloxy; $C_{5-7}$ cycloalkyl; halogen or nitro; and the physiologically acceptable salts thereof, which comprises:

(a) in the preparation of compounds of formula (2)

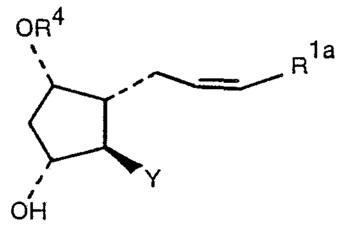

(2)

(wherein Y and $R^4$ are as defined for formula (1) and $R^{1a}$ is as defined for $R^1$ where $R^{10}$ is a hydrogen atom), reacting a compound of formula (3) or (3a)

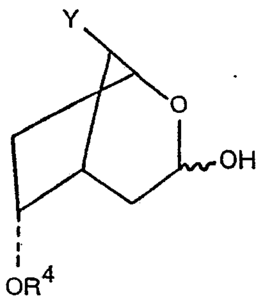 (3)

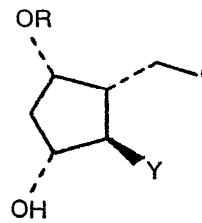 (3a)

with a phosphorane $R^{12}_3P = CHR^{1a}$ or a salt thereof (where $R^{12}$ is $C_{1-6}$ alkyl or phenyl);

(b) in the preparation of a compound in which A is (b), oxidising a compound of formula (1) in which A is (a)

(c) esterifying a compound of formula (1) in which $R^{10}$ is hydrogen to prepare a compound in which $R^{10}$ is alkyl or phenalkyl;

(d) saponifying a corresponding ester to prepare a compound of formula (1) in which $R^{10}$ is hydrogen;

(e) isomerising a compound of formula (1) in which A is the group (a) and X is cis —CH=CH— to prepare the corresponding trans compound;

(f) catalytically hydrogenating a compound of formula (1) in which X is —CH=CH— to produce a compound in which X is $—(CH_2)_2—$;

(g) etherifying a compound of formula (1) in which A is the group (a) and $—OR^4$ represents hydroxy, any other hydroxy group present being protected during the reaction;

(h) in the preparation of a compound of formula (1) in which A is (a), removing the protecting group from a corresponding compound in which the ring hydroxy group is protected;

(i) in the preparation of a compound of formula (1) in which $R^4$ is phenalkyl substituted by alkanoylamino, acylating the corresponding amine;

(j) in the preparation of a compound of formula (1) in which A is the group (a) and $R^4$ is phenalkyl substituted by alkylsulphinyl or alkylsulphonyl, oxidising the corresponding alkylthio compound;

(k) in the preparation of a compound as defined in claim 1 in which A is the group (a) and Y is a saturated heterocyclic amino group, treating the corresponding compound in which Y is $—NH_2$ with a difunctional reagent to form said heterocyclic amino group; or

(l) treating a compound of formula (1) with an acid or (where $R^{10}$ is hydrogen) a base to form a salt.

2. A process as claimed in claim 1 in which A in the product is the group (b);

3. A process as claimed in claim 1 or claim 2 in which Y in the product is morpholino, dioxothiamorpholino or piperidino.

4. A process as claimed in any one of the preceding claims in which X in the product is cis —CH=CH—.

5. A process as claimed in any one of the preceding claims in which $R^1$ in the product is $—(CH_2)_3COOR^{10}$ where $R^{10}$ is a hydrogen atom or methyl.

6. A process as claimed in any one of the preceding claims in which $R^4$ in the product is benzyl substituted by phenyl-($C_{1-3}$) alkyl or phenyl substituted by $C_{1-3}$ alkoxy or $C_{1-3}$ alkyl, or is thienylmethyl substituted by phenyl or ($C_{1-3}$ alkoxy) phenyl.

7. A process as claimed in claim 1 in which in the product,

A is the group (b),
X is cis —CH=CH;
$R^1$ is $—(CH_2)_3COOH$,
Y is morpholino, piperidino or dioxothiamorpholino, and
$R^4$ is benzyl substituted by phenethyl, benzyl, methoxyphenyl or methylphenyl, or is methoxyphenylthienylmethyl.

8. A process as claimed in any one of the preceding claims, other than those in which $R^4$ in the product is phenalkyl substituted by thienyl, substituted aminosulphonyl or substituted alkanoylaminosulphonyl.

9. A process for the manufacture of a pharmaceutical composition comprising a compound of formula (1) as defined in any one of the preceding claims, which comprises formulating said compound with one or more pharmaceutical carriers.

10. A process as claimed in claim 9 in which a unit dose of the compound of formula (1) is formulated as a tablet or liquid for injection, or in which the compound of formula (1) is formulated as an aerosol, solution or inhalation cartridge for the inhalation of a metered amount of said compound.

**Patensprüche für die Vertragsstaaten: BE CH DE FR GB IT LI NL SE**

1. Prostanoide der allgemeinen Formel (1)

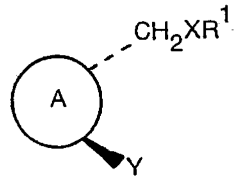

(1)

worin A bedeutet

(a) 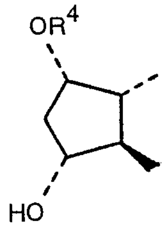 oder (b) 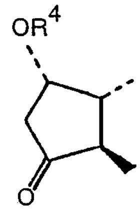

X ist cis oder trans —CH=CH— oder —$(CH_2)_2$—;

$R^1$ ist gerades oder verzweigtes $C_{1-7}$-Alkyl, das als Endsubstituenten —$COOR^{10}$ trägt, wobei $R^{10}$ ein Wasserstoffatom, $C_{1-6}$-Alkyl oder $C_{7-10}$-Phenalkyl ist;

Y ist eine gesättigte heterocyclische Aminogruppe mit 5 bis 8 Ringgliedern und (a) enthält gegebenenfalls im Ring —O—, —S—, —$SO_2$, —$NR^{14}$— (worin $R^{14}$ ein Wasserstoffatom, $C_{1-7}$-Alkyl oder Phenalkyl mit einem $C_{1-4}$-Alkylteil ist),

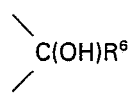

(worin $R^6$ ein Wasserstoffatom, $C_{1-7}$-Alkyl, Phenyl oder Phenalkyl mit einem $C_{1-4}$-Alkylteil ist); und/oder (b) ist gegebenenfalls substituiert durch eine oder mehrere $C_{1-4}$-Alkylgruppen;

$R^4$ ist (i) Phenalkyl (mit einem $C_{1-3}$-Alkylteil, wobei der Phenylteil substituiert ist durch $C_{1-3}$-Alkylthio, $C_{1-3}$-Alkylsulphinyl, $C_{1-3}$-Alkylsulphonyl, $C_{1-3}$-Alkanoylamino, Benzoylamino, Phenylalkyl mit einem $C_{1-3}$-Alkylteil, Aminosulphonyl (wobei die Amino-Gruppen gegebenenfalls durch eine oder zwei $C_{1-3}$-Alkylgruppen substituiert sind), $C_{1-3}$-Alkanoylaminosulphonyl (wobei die Aminogruppe gegebenenfalls durch $C_{1-3}$-Alkyl substituiert ist), Phenylsulphonyl (wobei der Phenylteil gegebenenfalls durch $C_{1-3}$-Alkyl substituiert ist), Nitro, Tetrazol-5-yl, Phenyl substituiert durch $R^5$ (wobei $R^5$ $C_{1-4}$-Alkyl, $C_{1-4}$-Alkoxy, Halogen oder Phenyl ist), oder Thienyl); oder (ii) die Gruppe :

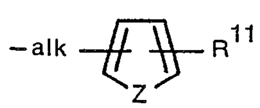

worin

Alk $C_{1-3}$-Alkylen ist;

Z ist O oder S;

$R^{11}$ ist ein Wasserstoffatom; $C_{1-6}$-Alkyl, $C_{1-6}$-Alkoxy, Phenyl oder Phenylalkoxy oder Phenylalkyl mit einem $C_{1-3}$-Alkylteil (der Phenyltiel ist im jeweiligen Fall gegebenenfalls substituiert durch $C_{1-3}$-Alkyl, $C_{1-3}$-Alkoxy oder Halogen), PHenyloxy, $C_{5-7}$-Cycloalkyl, Halogen oder Nitro; und die physiologisch annehmbaren Salze davon.

2. Verbindungen gemäs Anspruch 1, worin A die Gruppe (b) ist.

3. Verbindung gemäß Anspruch 1 oder 2, worin Y Morpholino, Dioxothiamorpholino oder Piperidino ist.

4. Verbindungen gemäß einem der vorhergehenden Ansprüche, worin X cis —CH=CH— ist.

5. Verbindung gemäß einem der vorhergehenden Ansprüche, worin $R^1$ —$(CH_2)_3COOR^{10}$ ist, worin $R^{10}$ ein Wasserstoffatom oder Methyl ist.

6. Verbindungen gemäß einem der vorhergehenden Ansprüche, worin $R^4$ Benzyl, substituiert durch Phenyl-($C_{1-3}$)-Alkyl oder Phenyl, substituiert durch $C_{1-3}$-Alkoxy oder $C_{1-3}$-Alkyl oder Thienylmethyl, substituiert durch Phenyl oder ($C_{1-3}$-Alkoxy)-Phenyl ist.

7. Verbindungen gemäß Anspruch 1, worin
A die Gruppe (b) ist,
X cis —CH=CH ist,
$R^1$ —$(CH_2)_3COOH$ ist,
Y Morpholino, Piperadino oder Dioxothiamorpholino ist und
$R^4$ Benzyl, substituiert durch Phenethyl, Benzyl, Methoxyphenyl oder Methylphenyl ist, oder Methoxyphenylthienylmethyl ist.

8. Verbindung gemäß einem der vorhergehenden Ansprüche anders als diejenigen, worin $R^4$ Phenalkyl substituiert durch Thienyl, substituiertes Aminosulphonyl oder substituiertes Alkanoylaminosulphonyl ist.

9. Eine pharmazeutische Zusammensetzung umfassend eine Verbindung, wie in einem der vorhergehenden Ansprüche beansprucht, zusammen mit einem oder mehreren pharmazeutischen Trägern.

10. Verfahren zur Herstellung einer Verbindung gemäß Anspruch 1, welchesd umfaßt:
a) Zur Herstellung von Verbindungen der Formel (2),

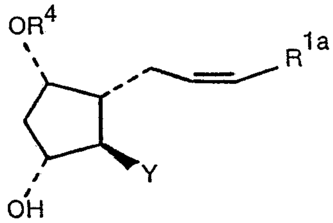

(2)

(worin Y und $R^4$ wie in Anspruch 1 definiert sind und $R^{1a}$ wie für $R^1$ definiert ist, wobei $R^{10}$ ein Wasserstoffatom ist), Reaktion einer Verbindung der Formel (3) oder (3a),

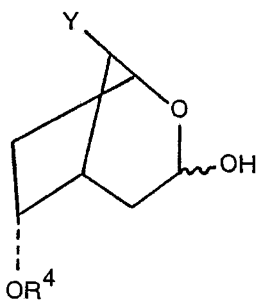

(3)

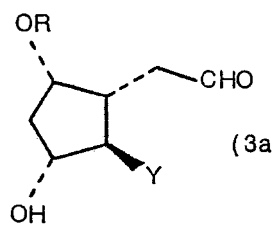

(3a)

mit einem Phosphoran $R_3^{12}P{=}CHR^{1a}$ oder einem Salz davon (worin $R^{12}$ $C_{1-6}$-Alkyl oder Phenyl ist);

b) zur Herstellung einer Verbindung, worin A (b) ist, Oxidieren einer Verbindung, wie in Anspruch 1 definiert, worin A = (a);

c) Verestern einer Verbindung wie in Anspruch 1 definiert, worin $R^{10}$ Wasserstoff ist, zur Herstellung einer Verbindung, worin $R^{10}$ Alkyl oder Phenalkyl ist;

d) Verseifen eines entsprechenden Esters zur Herstellung einer Verbindung, wie in Anspruch 1 definiert, worin $R^{10}$ Wasserstoff ist;

e) Isomerisieren einer Verbindung, wie in Anspruch 1 definiert, worin A die Gruppe (a) ist und X cis —CH=CH– ist, zur Herstellung der entsprechenden trans-Verbindung;

f) katalytisches Hydrieren einer Verbindung, wie in Anspruch 1 definiert, worin X —CH=CH— ist, zur Herstellung einer Verbindung, worin X —$(CH_2)_2$— ist;

g) Veräthern einer Verbindung, wie in Anspruch 1 definiert, worin A die Gruppe (a) ist und —$OR^4$ Hydroxy bedeutet, wobei irgendwelche anderen vorhandenen Hydroxy-Gruppen während der Reaktion geschützt sind;

h) zur Herstellung einer Verbindung, wie in Anspruch 1 definiert, worin A = (a), Entfernen der Schutzgruppe einer entsprechenden Verbindung, worin die Ringhydroxy-Gruppe geschützt ist;

i) zur Herstellung einer Verbindung, wie in Anspruch 1 definiert, worin $R^4$ Phenalkyl substituiert durch Alkanoylamino ist, Acylieren des entspechenden Amins;

j) zur Herstellung einer Verbindung, wie in Anspruch 1 definiert, worin A die Gruppe (a) ist, und $R^4$ Phenalkyl, substituiert durch Alkylsulphinyl oder Alkylsulphonyl ist, Oxidieren der entsprechenden Alkylthioverbindung;

k) zur Herstellung einer Verbindung, wie in Anspruch 1 definiert, worin A die Gruppe (a) ist, und Y eine gesättigte heterocyclische Aminogruppe ist, Behandeln der entsprechenden Verbindung, worin Y —$NH_2$ ist, mit einem difunktionellen Reagens zur Bildung der genannten heterocyclischen Aminogruppe; oder

l) Behandeln einer Verbindung, wie in Anspruch 1 definiert, mit einer Säure oder (wenn $R^{10}$ Wasserstoff ist) einer Base zur Bildung eines Salzes.

**Patentansprüche für den Vertragsstaat: AT**

1. Verfahren zur Herstellung von Prostanoiden der allgemeinen Formel (1)

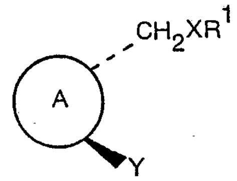

(1)

worin A bedeutet a oder b,

(a) 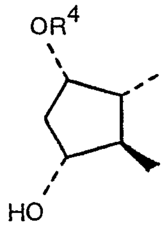 oder (b) 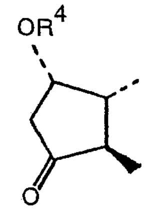

X ist cis oder trans —CH=CH— oder —$(CH_2)_2$—;

$R^1$ ist gerades oder verzweigtes $C_{1-7}$-Alkyl, tragend als Endsubstituenten —$COOR^{10}$, worin $R^{10}$ ein Wasserstoffatom, $C_{1-6}$-Alkyl oder $C_{7-10}$-Phenalkyl ist;

Y ist eine gesättigte heterocyclische Aminogruppe mit 5 bis 8 Ringgliedern und (a) enthält gegebenenfalls im Ring —O—, —S—, —$SO_2$, —$NR^{14}$— (worin $R^{14}$ ein Wasserstoffatom, $C_{1-7}$-Alkyl oder Phenalkyl mit einem $C_{1-4}$-Alkylteil ist),

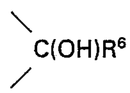

(worin $R^6$ ein Wasserstoffatom, $C_{1-7}$-Alkyl, Phenyl oder Phenalkyl mit einem $C_{1-4}$-Alkylteil ist); und/oder (b) ist gegebenenfalls substituiert durch eine oder mehrere $C_{1-4}$-Alkylgruppen;

$R^4$ ist (i) Phenalkyl (mit einem $C_{1-3}$-Alkylteil und der Phenylteil ist substituiert durch $C_{1-3}$-Alkylthio, $C_{1-3}$-Alkylsulphinyl, $C_{1-3}$-Alkylsulphonyl, $C_{1-3}$-Alkanoylamino, Benzoylamino, Phenylalkyl mit einem $C_{1-3}$-Alkylteil, Aminosulphonyl (wobei die Amino-Gruppen gegebenenfalls substituiert ist durch eine oder zwei $C_{1-3}$-Alkylgruppen), $C_{1-3}$-Alkanoylaminosulphonyl (wobie die Aminogruppe gegebenenfalls substituiert ist durch $C_{1-3}$-Alkyl), Phenylsulphonyl (wobei der Phenylteil gegebenenfalls substituiert ist durch $C_{1-3}$-Alkyl), Nitro, Tetrazol-5-yl, Phenyl substituiert durch $R^5$ (wobei $R^5$ $C_{1-4}$-Alkyl, $C_{1-4}$-Alkoxy, Halogen oder Phenyl ist), oder Thienyl); oder (ii) die Gruppe :

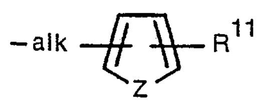

worin

alk $C_{1-3}$-Alkylen ist;

Z ist O oder S;

$R^{11}$ ist ein Wasserstoffatom; $C_{1-6}$-Alkyl, $C_{1-6}$-Alkoxy, Phenyl oder Phenylalkoxy oder Phenylalkyl mit einem $C_{1-3}$-Alkylteil (wobei der Phenyltiel ist im jeweiligen Fall gegebenenfalls substituiert ist durch $C_{1-3}$-Alkyl, $C_{1-3}$-Alkoxy oder Halogen), Phenyloxy, $C_{5-7}$-Cycloalkyl, Halogen oder Nitro; und der physiologisch annehmbaren Salze davon, welches umfaßt:

a) Zur Herstellung von Verbindungen der Formel (2),

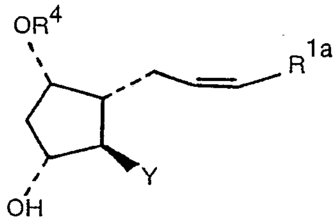

(2)

(worin Y und $R^4$ wie für Formel (1) defniert sind und $R^{1a}$ wie für $R^1$ definiert ist, wobei $R^{10}$ ein Wasserstoffatom ist), Reaktion einer Verbindung der Formel (3) oder (3a),

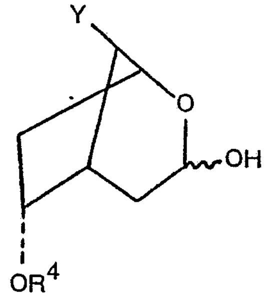 (3) 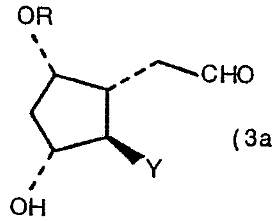 (3a)

mit einem Phosphoran $R_3^{12}P=CHR^{1a}$ oder einem Salz davon (worin $R^{12}$ $C_{1-6}$-Alkyl oder Phenyl ist);

b) zur Herstellung einer Verbindung, worin A die Bedeutung (b) hat, Oxidieren einer Verbindung der Formel 1, worin A die Bedeutung (a) hat;

c) Verestern einer Verbindung der Formel 1, worin $R^{10}$ Wasserstoff ist, zur Herstellung einer Verbindung, worin $R^{10}$ Alkyl oder Phenalkyl ist;

d) Verseifen eines entsprechenden Esters zur Herstellung einer Verbindung der Formel 1, worin $R^{10}$ Wasserstoff ist;

e) Isomerisieren einer Verbindung der Formel 1, worin A die Gruppe (a) ist und X cis $-CH=CH-$ ist, zur Herstellung der entsprechenden trans-Verbindung;

f) katalytisches Hydrieren einer Verbindung der Formel 1, worin X $-CH=CH-$ ist, zur Herstellung einer Verbindung, worin X $-(CH_2)_2-$ ist;

g) Veräthern einer Verbindung der Formel 1, worin A die Gruppe (a) ist und $-OR^4$ Hydroxy bedeutet, wobei irgendwelche anderen vorhandenen Hydroxy-Gruppen während der Reaktion geschützt sind;

h) zur Herstellung einer Verbindung der Formel 1, worin A die Bedeutung (a) hat, Entfernen der Schutzgruppe aus einer entsprechenden Verbindung, worin die Ringhydroxy-Gruppe geschützt ist;

i) zur Herstellung einer Verbindung der Formel 1, worin $R^4$ Phenalkyl substituiert durch Alkanoylamino ist, Acylieren des entspechenden Amins;

j) zur Herstellung einer Verbindung der Formel 1, worin A die Gruppe (a) ist, und $R^4$ Phenalkyl substituiert durch Alkylsulphinyl oder Alkylsulphonyl ist, Oxidieren der entsprechenden Alkylthioverbindung;

k) zur Herstellung einer Verbindung, wie in Anspruch 1 definiert, worin A die Gruppe (a) ist, und Y eine gesättigte heterocyclische Aminogruppe ist, Behandeln der entsprechenden Verbindung, worin Y $-NH_2$ ist, mit einem difunktionellen Reagens zur Bildung der genannten heterocyclischen Amino-gruppe; oder

l) Behandlen einer Verbindung durch Formel 1 mit einer Säure oder (wenn $R^{10}$ Wasserstoff ist) einer Base zur Bildung eines Salzes.

2. Verbindungen gemäß Anspruch 1, dadurch gekennzeichnet, daß A in dem Produkt die Gruppe (b) ist.

3. Verfahren gemäß Anspruch 1 oder Anspruch 2 dadurch gekennzeichnet, daß Y in dem Produkt Morpholinio, Dioxothiamorpholino oder Piperidino ist.

4. Verfahren gemäß einem der vorhergehenden Ansprüche, worin X in dem Produkt cis $-CH=CH-$ ist.

5. Verfahren gemäß einem der vorhergehenden Ansprüche worin $R^1$ in dem Produkt $-(CH_2)_3COOR^{10}$ ist, wobei $R^{10}$ ein Wasserstoffatom oder $C_{1-4}$-Alkyl ist.

6. Verfahren gemäß einem der vorhergehenden Ansprüche, worin $R^4$ in dem Produkt Benzyl, substituiert durch Phenyl-($C_{1-3}$)-Alkyl oder Phenyl, substituiert durch $C_{1-3}$-Alkoxy oder $C_{1-3}$-Alkyl ist, oder Thienylmethyl, substituiert durch Phenyl oder ($C_{1-3}$-Alkoxy)-Phenyl ist.

7. Verfahren gemäß Anspruch 1, worin in dem Produkt

A die Gruppe (b)ist,

X cis $-CH=CH$ ist,

$R^1$ $-(CH_2)_3$ COOH ist,

Y Morpholino, Piperidino oder Dioxothiamorpholino, und

$R^4$ Benzyl, substituiert durch Phenethyl, Benzyl, Methoxyphenyl oder Methylphenyl ist, oder Methoxyphenylthienylmethyl ist.

8. Verfahren gemäß einem der vorhergehenden Ansprüche, anders als solche, worin $R^4$ in dem Produkt Phenalkyl, substituiert durch Thienyl, substituiertes Aminosulphonyl oder substituiertes Alkanoylaminosulphonyl ist.

9. Verfahren zur Herstellung einer pharmazeutischen Zusammensetzung, umfassend eine Verbindung der Formel (1), wie in irgendeinem der vorhergehenden Ansprüche definiert, welches umfaßt das Formulieren dieser Verbindung mit einem oder mehreren pharmazeutischen Trägern.

10. Verfahren gemäß Anspruch 9, dadurch gekennzeichnet, daß eine Einheitsdosis der Verbindung der Formel (1) als Tablette oder Flüssigkeit zur Injektion formuliert wird, oder die Verbindung der Formel (1) als Aerosol, Lösung oder Inhalationspatrone zum Inhalieren einer abgemessenen Menge der Verbindung formuliert ist.

**Revendications pour les Etats contractants: BE CH DE FR GB IT LI NL SE**

1. Prostanoïdes de formule développée (1)

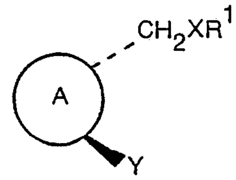

(1)

dans laquelle

A représente

(a) 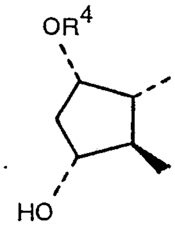 ou (b) 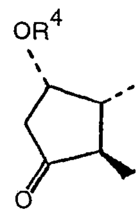

X est —CH=CH— ou —$(CH_2)_2$— cis ou trans;

$R^1$ est un alcoyle linéaire ou ramifié en $C_1$ à $C_7$ portant comme substituant terminal —$COOR^{10}$, $R^{10}$ étant un atome d'hydrogène, alcoyle en $C_1$ à $C_6$ ou phénylalcoyle en $C_7$ à $C_{10}$;

Y est un groupe amino hétérocylcique saturé qui a de 5 à 8 chaînons, et (a) contient éventuellement dans le noyau —O—, —S—, —$SO_2$, —$NR^{14}$— ($R^{14}$ étant un atome d'hydrogène, alcoyle en $C_1$ à $C_7$, ou phénylalcoyle, ayant une portion alcoyle en $C_1$ à $C_4$),

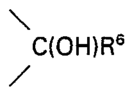

($R^6$ étant un atome d'hydrogène, alcoyle en $C_1$ à $C_7$, phényle ou phénylalcoyle ayant une portion alcoyle en $C_1$ à $C_4$,); et/ou (b) est éventuellement substitué par un ou par plusieurs groupes alcoyle en $C_1$ à $C_4$;

$R^4$ est (i) phénylalcoyle (ayant une portion alcoyle en $C_1$à $C_3$ et la portion phényle étant substituée par un alcoylthio en $C_1$à $C_3$, un alcoylsulfinyle en $C_1$ à $C_3$, un alcoylsulfonyle en $C_1$ à $C_3$, un alcanoylamino en $C_1$ à $C_3$, un benzoylamino, un phénylalcoyle ayant une portion alcoyle en $C_1$ à $C_3$, un aminosulfonyle (le groupe amino étant éventuellement substitué par un ou par deux groupes alcoyle en $C_1$ à $C_3$), un alcanoylamino-sulfonyl en $C_1$ à $C_3$ (le groupe amino étant éventuellement substitué par un alcoyle en $C_1$ à $C_3$), un phényl-sulfonyle (la portion phényle étant éventuellement substituée par un alcoyle en $C_1$ à $C_3$), un groupe nitro, un tétrazol-5-yle, un phényle substitué par $R^5$ ($R^5$ étant alcoyle en $C_1$ à $C_4$, alcoxy en $C_1$ à $C_4$, halogène ou phényle), ou un thiényle);

ou (ii) le groupe:

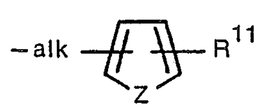

dans laquelle

alk est alcoylène en $C_1$ à $C_3$;

Z est O, ou S;

$R^{11}$ est un atome d'hydrogène; un alcoyle en $C_1$à $C_6$; un alcoxy en $C_1$ à $C_6$; un phényle ou un phényl-alcoxy ou un phénylalcoyle ayant une portion alcoyle en $C_1$à $C_3$ (la portion phényle étant dans chaque cas éventuellement substituée par un alcoyle en $C_1$ à $C_3$, un alcoxy en $C_1$ à $C_3$ ou un halogène); un phényloxy; un cycloalcoyle en $C_5$ à $C_7$; un halogène ou un groupe nitro; et leurs sels acceptables physiologiquement.

2. Composés tels que revendiqués à la revendication 1, dans lesquels A est le groupe (b).

3. Composés tels que revendiqués à la revendication 1 ou 2, dans lesquelles Y est morpholino, dioxothiamorpholino ou pipéidino.

4. Composés tels que revendiqués à l'une quelconque des revendications précédentes, dans lesquels X est —CH=CH— cis.

5. Composés tels que revendiqués suivant l'une quelconque des revendications précédentes, dans lesquels $R^1$ est —$(CH_2)_3COOR^{10}$, $R^{10}$ étant un atome d'hydrogène ou méthyle.

6. Composés tels que revendiqués suivant l'une quelconque des revendications précédentes, dans lesquels $R^4$ est benzyle substitué par un phénylalcoyle ayant de 1 à 3 atomes de carbone dans la portion alcoyle, ou phényle substitué par un alcoxy en $C_1$ à $C_3$ ou par un alcoyle en $C_1$ à $C_3$, ou est thénylméthyle substitué par un phényle ou par un alcoxyphényle ayant de 1 à 3 atomes de carbone dans la portion alcoxy.

7. Composés tels que revendiqués à la revendication 1, dans lesquels:
A est le groupe (b),
X est —CH=CH— cis,
$R^1$ est $—(CH_2)_3COOH$,
Y est morpholino, pipéridino ou dioxothiamorpholino, et
$R^4$ est benzyle substitué par un phénéthyle, un benzyle, un méthoxyphényle ou un méthylphényle, ou est méthoxyphénylthiénylméthyle.

8. Composés tels que revendiqués suivant l'une quelconque des revendications précédentes, autres que ceux dans lesquels $R^4$ est phénylalcoyle substitué par un thiényle, un aminosulfonyl substitué ou un alcanoylaminosulfonyl substitué.

9. Composition pharmaceutique comprenant un composé tel que revendiqué dans l'une quelconque des revendications précédentes, associé à une à plusieurs véhicles pharmaceutiques.

10. Procédé de préparation d'un composé tel que revendiqué à la revendication 1 qui comprend:
(a) dans la préparation de composés de formule (2)

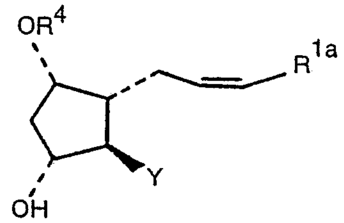
(2)

(dans laquelle Y et $R^4$ sont tels que définis à la revendication 1 et $R^{1a}$ est tel que défini pour $R^1$ quand $R^{10}$ est un atome d'hydrogène), la réaction d'un composé de formule (3) ou (3a)

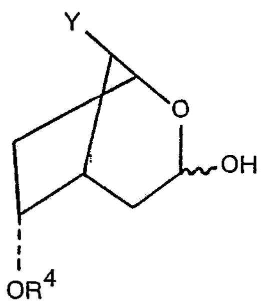
(3)

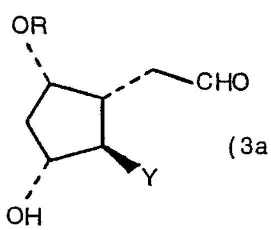
(3a)

sur un phosphorane $R_3^{12}p{=}CHR^{1a}$ ou sur l'un de des sels ($R^{12}$ étant alcoyle en $C_1$ à $C_6$ ou phényle);

(b) dans la préparation d'un composé dans lequel A est (b), l'oxydation d'un composé tel que défini à la revendication 1 dans laquelle A est (a)

(c) l'estérification d'un composé tel que défini à la revendication 1, dans lequel $R^{10}$ est l'hydrogène, pour préparer un composé dans lequel $R^{10}$ est alcoyle ou phénylalcoyle;

(d) la saponification d'un ester correspondant pour préparer un composé tel que défini à la revendication 1, dans lequel $R^{10}$ est l'hydrogène;

(e) l'isomérisation d'un composé tel que défini à la revendication 1, dans lequel A est le groupe (a) et X est —CH=CH— cis pour préparer le composé trans correspondant;

(f) l'hydrogenation catalytique d'un composé tel que défini à la revendication 1 dans lequel X est —CH=CH— pour préparer un composé dans lequel X est $—(CH_2)_2—$;

(g) l'éthérification d'un composé tel que défini à la revendication 1 dans lequel A est le groupe (a) et $—OR^4$ représente un groupe hydroxy, tout autre groupe hydroxy présent étant protégé pendant la réaction;

(h) dans la préparation d'un composé tel que défini à la revendication 1 dans laquelle A est (a), l'enlévement du groupe de protection d'un composé correspondant dans lequel le groupe hydroxy du cycle est protégé;

(i) dans la préparation d'un composé tel que défini à la revendication 1, dans lequel $R^4$ est phénylalcoyle substitué par un alcanoylamino, l'acylation de l'amine correspondante;

(j) dans la préparation d'un composé tel que défini à la revendication 1, dans lequel A est le groupe (a) et $R^4$ est phénylalcoyle substitué par un alcoylsulfinyle ou par un alcoylsulfonyle, l'oxydation du composé alcoylthio correspondant;

(k) dans la préparation d'un composé tel que défini à la revendication 1, dans lequel A est le groupe (a) et Y est un groupe amino hétérocyclique saturé, le traitement du composé correspondant dans lequel Y est $—NH_2$ par un réactif difonctionnel pour former le groupe amino hétérocyclique; ou

(l) le traitement d'un composé tel que défini à la revendication 1 par un acide ou (quand $R^{10}$ est l'hydrogène) par une base pour former un sel.

**Revendications pour L'Etat contractant: AT**

1. Procédé de préparation de prostanoïdes de formule développée (1)

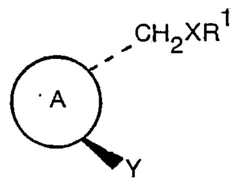

(1)

dans laquelle

A représente

(a)

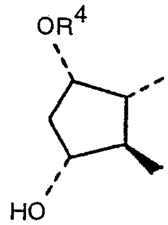

ou

(b)

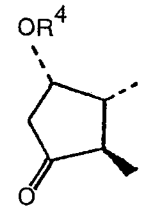

X est —CH=CH— ou $—(CH_2)_2—$ cis ou trans;

$R^1$ est un alcoyle linéaire ou ramifié en $C_2$ à $C_7$ portant comme substituant terminal $—COOR^{10}$ $R^{10}$ étant un atome d'hydrogène, alcoyle en $C_1$à $C_6$, ou phénylalcoyle en $C_7$ à $C_{10}$;

Y est un groupe amino hétérocyclique saturé qui a de 5 à 8 chaînons, et (a) contient éventuellement dans le noyau —O—, —S—, $—SO_2$, $—NR^{14}—$ ($R^{14}$ étant un atome d'hydrogène, alcoyle en $C_1$ à $C_7$, ou phénylalcoyle, ayant une portion alcoyle en C à $C_4$),

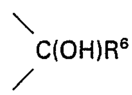

($R^6$ étant un atome d'hydrogène, alcoyle en $C_1$ à $C_7$, phényle ou phénylalcoyle ayant une portion alcoyle en $C_1$ à $C_4$); et/ou (b) est éventuellement substitué par un ou plusieurs groupes alcoyle en $C_1$ à $C_4$;

$R^4$ est (i) phénylalcoyle (ayant une portion alcoyle en $C_1$ à $C_3$ et la portion phényle étant substituée par un alcoylthio en $C_1$ à $C_3$, un alcoylsulfinyl en $C_1$ à $C_3$, un alcoylsulfonyl en $C_1$ à $C_3$, un alcanoylamino en $C_1$ à $C_3$, un benzoylamino, un phénylalcoyle ayant une portion alcoyle en $C_1$ à $C_3$, un aminofulfonyle (le groupe amino étant éventuellement substitué par un ou par deux groupes alcoyle en $C_1$ à $C_3$), un alcanoylamino-sulfonyle en $C_1$ à $C_3$ (le groupe amino étant éventuellement substitué par un alcoyle en $C_1$ à $C_3$), un phényl-sulfonyle (la portion phényle étant éventuellement substituée par un alcoyle en $C_1$ à $C_3$), un groupe nitro, un tétrazol-5-yle, un phényl substitué par $R^5$ ($R^5$ étant alcoyle en $C_1$ à $C_4$, alcoxy en $C_1$ à $C_4$, halogène ou phényle), ou un thiényle);

ou (ii) le groupe:

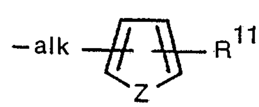

dans laquelle

alk est alcoylène en $C_1$ à $C_3$;

Z est O, ou S;

$R^{11}$ est un atome d'hydrogène; un alcoyle en $C_1$ à $C_6$; un alcoxy en $C_1$ à $C_6$; un phényle ou un phényl-alcoxy ou un phénylalcoyle ayant une portion alcoyle en $C_1$ à $C_3$ (la portion phényle étant dans chaque cas éventuellement substituée par un alcoyle en $C_1$ à $C_3$, un alcoxy en $C_1$ à $C_3$ ou un halogène); un phényloxy; un cycloalcoyle en $C_5$ à $C_7$; un halogène ou un groupe nitro; et de leurs sels acceptables physiologiquement, qui comprend:

(a) dans la préparation de composés de formule (2)

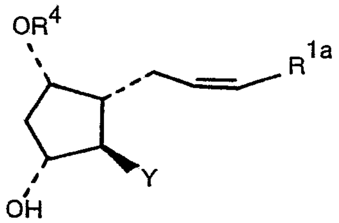

(2)

(dans laquelle Y et $R^4$ sont tels que définis par la formule 1 et $R^{1a}$ est tel que défini pour $R^1$ quand $R^{10}$ est un atome d'hydrogène), la réaction d'un composé de formule (3) ou (3a)

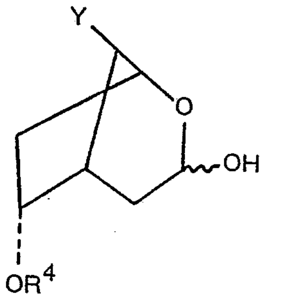

(3)

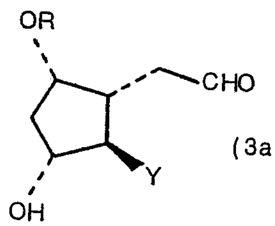

(3a)

sur un phosphorane $R_3^{12}p{=}CHR^{1a}$ ou sur l'un de des sels ($R^{12}$ étant alcoyle en $C_1$à $C_6$ ou phényle);

(b) dans la préparation d'un composé dans lequel A est (b), l'oxydation d'un composé de formule 1 dans laquelle A est (a);

(c) l'estérification d'un composé de formule 1, dans lequel $R^{10}$ est l'hydrogène; pour préparer un composé dans lequel $R^{10}$ est alcoyle ou phénylalcoyle;

(d) la saponification d'un ester correspondant pour préparer un composé de formule 1, dans lequel $R^{10}$ est l'hydrogène;

(e) l'isomérisation d'un composé de formule 1, dans lequel A est le groupe (a) et X est —CH=CH— cis pour préparer le composé trans correspondant;

(f) l'hydrogénation catalytique d'un composé de formule 1, dans lequel X est —CH=CH—, pour préparer un composé dans lequel X est $-(CH_2)_2-$;

(g) l'éthérification d'un composé de formule 1, dans lequel A est le groupe (a) et $-OR^4$ représente un groupe hydroxy, toute autre groupe hydroxy présent étant protégé pendant la réaction;

(h) dans la préparation d'un composé de formule 1, dans lequel A est (a), l'enlèvement du groupe de protection d'un composé correspondant dans lequel le groupe hydroxy du cycle est protégé;

(i) dans la préparation d'un composé de formule 1, dans lequel $R^4$ est phénylalcoyle substitué par un alcanoylamino, l'acylation de l'amine correspondante;

(j) dans la préparation d'un composé de formule 1, dans lequel A est le groupe (a) et $R^4$ est phénylalcoyle substitué par un alcoylsulfinyle ou par un alcoylesulfonyle, l'oxydation du composé alcoylthio correspondant;

(k) dans la préparation d'un composé de formule 1, dans lequel A est le groupe (a) et Y est un groupe amino hétérocyclique saturé, le traitement du composé correspondant dans lequel Y est $-NH_2$ par un réactif difonctionnel pour former le groupe amino hétérocyclique; ou

(l) le traitement d'un composé de formule 1, par un acide ou (quand $R^{10}$ est l'hydrogène) par une base pour former un sel.

2. Procédé tel que revendiqué à la revendication 1, dans lequel A dans le produit est le groupe (b).

3. Procédé tel que revendiqué à la revendication 1 ou à la revendication 2, dans lequel Y dans le produit est morpholino, dioxothiamorpholino ou pipéridino.

4. Procédé tel que revendiqué à l'une quelconque des revendications précédentes, dans lequel X dans le produit est —CH=CH— cis.

5. Procédé tel que revendiqué suivant l'une quelconque des revendications précédentes, dans lequel $R^1$ dans le produit est $-(CH_2)_3COOR^{10}$, $R^{10}$étant un atome d'hydrogène ou méthyle.

6. Procédé tel que revendiqué suivant l'une quelconque des revendications précédentes, dans lequel $R^4$ dans le produit est benzyle substitué par un phénylalcoyle ayant de 1 à 3 atomes de carbone dans la portion alcoyle ou phényle substitué par un alcoxy en $C_1$ à $C_3$ ou par un alcoyle en $C_1$ à $C_3$ ou est thiénylméthyl substitué par un phényle ou par un alcoxyphényle ayant de 1 à 3 atomes de carbone dans la portion alcoxy.

7. Procédé tel que revendiqué à la revendication 1, dans lequel dans le produit,

A est le groupe (b),

X est —CH=CH— cis,

$R^1$ est $-(CH_2)_3COOH$,

Y est morpholino, pipéridino ou dioxothiamorpholino, et

$R^4$ est benzyle substitué par un phénéthyle, un benzyle, un méthoxyphényle ou un méthylphényle, ou est méthoxyphénylthiénylméthyle.

8. Procédé tel que revendiqué à l'une quelconque des revendications précédentes autre que celui dans lequel $R^4$ dans le produit est phénylalcoyle substitué par un thiényle, un aminosulfonyle substitué ou un alcanoylaminosulfonyle substitué.

9. Procédé de fabrication d'une composition pharmaceutique comprenant un composé de formule (1) tel que défini à l'une quelconque des revendications précédentes, qui consiste à formuler ce composé avec un ou plusieurs véhicules pharmaceutiques.

10. Procédé tel que revendiqué à la revendication 9, dans lequel on formule une dose unitaire du composé de formule (1) sous la forme d'un comprimé ou d'un liquide d'injection, ou dans lequel on formule le composé de formule (1) en aérosol, en solutions ou en cartouche d'inhalation pour l'inhalation d'une quantité mesurée du composé.